(19) Europäisches Patentamt
European Patent Office
Office européen des brevets

(11) Publication number: **0 025 602**
**B1**

# (12) EUROPEAN PATENT SPECIFICATION

(45) Date of publication of patent specification: **19.12.84**

(51) Int. Cl.³: **C 07 D 471/04,** A 61 K 31/435

(21) Application number: **80105492.5**

(22) Date of filing: **12.09.80**

## (54) Acylated cephalosporin analogs, process for their preparation and pharmaceutical compositions comprising them.

(30) Priority: **13.09.79 JP 116720/79**
**25.10.79 JP 136987/79**
**23.02.80 JP 22035/80**

(43) Date of publication of application:
**25.03.81 Bulletin 81/12**

(45) Publication of the grant of the patent:
**19.12.84 Bulletin 84/51**

(84) Designated Contracting States:
**BE CH DE FR GB IT LI NL SE**

(56) References cited:
**BE-A- 875 053**
**BE-A- 875 054**
**DE-A-2 740 280**
**GB-A-1 538 240**

**The file contains technical information submitted after the application was filed and not included in this specification**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Hirata, Tadashi**
**1566-315, Nara-machi Midori-ku**
**Yokohama-shi Kanagawa-ken (JP)**
Inventor: **Ogasa, Takehiro**
**3-6-6, Asahi-machi Machida-shi**
**Tokyo (JP)**
Inventor: **Kobayashi, Shigeru**
**71-9, Kiso-machi Machida-shi**
**Tokyo (JP)**
Inventor: **Sato, Kiyoshi**
**326, Fushimi Shimizu-cho Sunto-gun**
**Shizuoka-ken (JP)**
Inventor: **Urakawa, Chikahiro**
**1384, Kiso-machi Machida-shi**
**Tokyo (JP)**
Inventor: **Sato, Akira**
**1880-30, Kiso-machi Machida-shi**
**Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Hashimoto, Yukio**
**3-5-15, Chuorinkan Yamato-shi**
**Kanagawa-ken (JP)**
Inventor: **Takasawa, Seigo**
**125-5, Minamiyana Hadano-shi**
**Kanagawa-ken (JP)**

(74) Representative: **Vossius Vossius Tauchner**
**Heunemann Rauh**
**Siebertstrasse 4 P.O. Box 86 07 67**
**D-8000 München 86 (DE)**

**Description**

Background of the Invention

The present invention relates to cephalosporin analogs represented by the general formula (I)

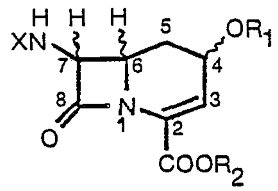

(I)

wherein X represents an acyl group, $R_1$ represents hydrogen or a lower alkyl group and $R_2$ represents hydrogen or a protective group of carboxylic acid and the pharmaceutically acceptable salts thereof.

The present invention contains optically active compounds of cephalosporin analogs represented by the general formula (I) and more particularly, the present invention contains optically active compounds of cephalosporin analogs represented by the general formula (II)

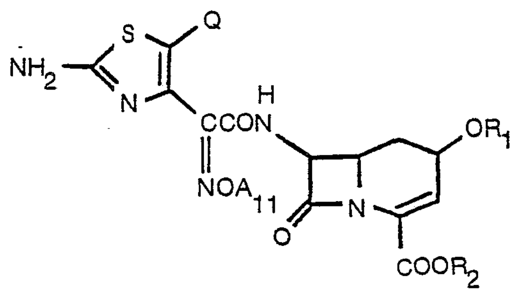

(II)

wherein Q represents hydrogen or a halo group (i.e. halogen atom), $A_{11}$ represents hydrogen or a substituted or unsubstituted alkyl, alkenyl, alkinyl, cycloalkyl, aryl or acyl group, $R_1$ and $R_2$ have the same significance as defined above, the hydrogens at the 6- and 7-positions have cis configuration and $OA_{11}$ group has syn configuration and pharmaceutically acceptable salts thereof.

Heretofore, a carbacephem compound, which is named according to the nomenclature in J. Am. Chem. Soc. *96*, 7584 (1974), wherein the sulfur atom of cephalosporin is substituted with a carbon atom and which has a substituted methyl group at the 3-position is described in the above reference and J. Med. Chem. *20*, 551 (1977). However, no compound having strong antibacterial activity has been reported. In Japanese Published Unexamined Patent Application No. 9296/79 (DE—A—2716707), compounds represented by the general formula (I) wherein the group $—OR_1$ is replaced by a hydrogen are mentioned but practical embodiment about examples for preparing the compounds and antibacterial activities thereof are not described in the reference.

The present inventors have succeeded in preparing carbacephem compounds having various substituents at the 4-, 5- or 3-position. The compounds are disclosed in the specifications of Japanese Published Unexamined Patent Application Nos. 128591/79, 49376/80 and 59186/80 and Japanese Patent Application Nos. 162005/78 and 8408/79 (DE—A—2911787, GB—A—2017103, etc.)

Furthermore, the present inventors have succeeded in preparing novel acylated carbacephems which are new antibiotics having strong antibacterial activities. The compounds are disclosed in the specifications of Japanese Published Unexamined Patent Application Nos. 128591/79, 49375/80, 53290/80 and 59185/80 and Japanese Patent Application Nos. 162006/78, 162007/78, 162008/78, 8409/79 and 92035/79 (DE—A—2911787, GB—A—2017103, etc;)

Hereinafter, compounds represented by the formula (I), (II), (III), . . . are named Compound [I], Compound [II], Compound [III], . . ., respectively.

Cephalosporin analogs in the specifications mentioned above are prepared by totally synthetic methods using optically inactive starting compounds, and they are optically inactive d1 compounds except a compound having a certain optically active acyl group, for example, D-phenylglycine, disclosed in the specification of Japanese Patent Application No. 162006/78. DE—A—2911787, GB—A—2017103, etc.).

As a result of various studies, the present inventors have succeeded in preparing optically active cephalosporin analogs which are disclosed in Japanese Patent Application No. 14534/79 filed by the present Applicant. [European Patent Application (referred to as E.P.A. hereinafter) 80100666.9, Publ.

No. 14476]. That is, the present inventors have found that optically active Compound [III] represented by the general formula (III).

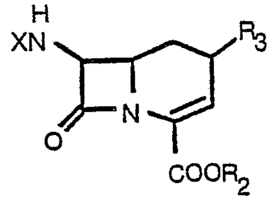

(III)

[wherein X and $R_2$ have the same significance as defined above and $R_3$ represent a hydrogen, a lower alkyl group or a lower acyloxy group], i.e. optically active acyl compounds prepared by using optically active compounds of the cephalosporin analogs represented by the general formula (IV)

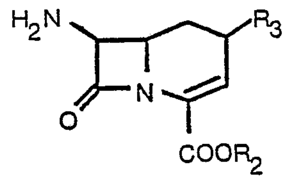

(IV)

(wherein $R_2$ and $R_3$ have the same significance as defined above and hydrogens at the 6- and 7-positions have cis configuration) have unexpectedly stronger antibacterial activity, i.e. 2 to 4 times of activity against various Gram-positive and Gram-negative microorganisms compared with the corresponding optically inactive dl-Compound [III].

Optically inactive d1 compounds corresponding to Compound [IV] are present as a mixture of equal amounts of the mirror image compounds represented by the general formulae (IV-1) and (IV-2)

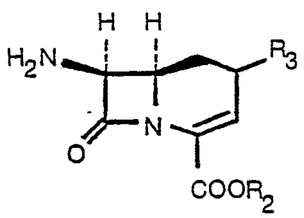

(IV–1)

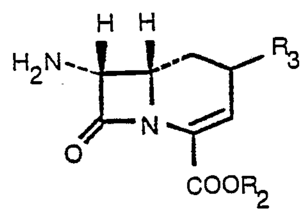

(IV–2)

(wherein $R_2$ and $R_3$ have the same significance as defined above).

The optically active compounds described in Japanese Patent Application Nos. 14533/79 and 14534/79 (E.P.A. 80100663.6 and 80100666.9; Publ. Nos. 14475 and 14476 resp.) are assumed to have the absolute structure represented by the general formula (IV-1) from various properties, stronger antimicrobial activity of the acyl compounds compared with that of the corresponding optically inactive dl-compound and the relationship between the absolute structure of cephalosporins and activities thereof.

In the above specification, the optically active compound is explained as having the absolute configuration 7$\alpha$-H and 6$\alpha$-H (6R 7S) corresponding to the general formula (IV-1). Compounds in the Examples and Reference Examples are also named according to the assumed absolute structural formula. Further, it is disclosed that optically active compounds are more useful as medicines or antimicrobial agents, than optically inactive compounds.

BE—A—875 054 discloses antibiotic cephalosporin analogs of the formula

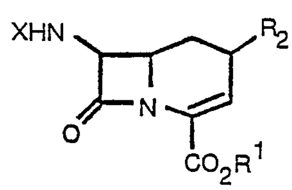

where X is acyl, $R^1$ is H or an esterifying group and $R^2$ is H, alkyl or acyloxy.

BE—A—875 053 discloses cephalosporin analogs useful in the preparation of cephalosporin type antibiotics, being compounds of the formula

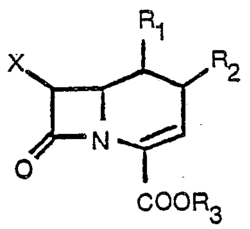

where X is $NH_2$, $N_3$ or phthalylimino; $R_1$ is H, halogen, OH or alkoxy, aryloxy, aralkyloxy, acyloxy, sulfonyloxy, alkylthio, arylthio, aralkylthio, alkylsulfinyl, arylsulfinyl, aralkylsulfinyl sulfonium, quaternary ammonium, alkylsulfonyl, arylsulfonyl, aralkylsulfonyl, arylselenyl or arylseleninyl; $R_2$ is defined for $R_1$ plus alkyl, haloalkyl, $N_3$, nitrile, amino or substituted amino; and $R_3$ is H, or substituted or unsubstituted alkyl, aryl, aralkyl or silyl. Acid addition salts where X is $NH_2$ and acid salts where $R_3$ is H are also disclosed.

DE—A—2 740 280 discloses 7-acylamino-8-oxo-3-oxa-1-azabicyclo[4,2,0]octan-2-carboxylic acids represented by the formula

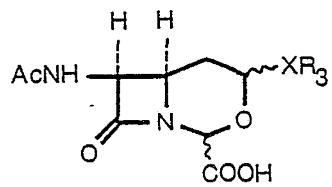

where X is S or O, $R_3$ is saturated or unsaturated, substituted or unsubstituted alkyl having 1 to 4 carbon atoms.

GB—A—1 538 240 discloses the stereoselective total synthesis of certain substituted $\Delta^{2,3}$-1,4-morpholine-2-carboxylic acids possessing a fused $\beta$-lactam ring in the 1,6-position and carrying a substituent cis to carbon 5 in the 7-position of the fused ring system represented by the general formula

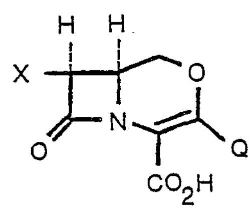

wherein Q is hydrogen, alkyl, aralkyl or —$CH_2COOZ$ where Z is hydrogen or the residue of an ester group and X is azido, amino or acylamino. When X is acylamino, these acids (and their pharmaceutically acceptable salts and physiologically hydrolyzed esters) are potent antibacterial agents.

The compounds of the present invention are different from those of BE—A—875 054 in the definition of the substituents ($OR_1$ and $R_2$) at the 4-position. The compounds of the present invention are different from those of BE—A—875 053 in the definition of the substituents ($OR_1$ and $R_2$, XNH and X) at the 4- and 7-positions. The compounds of the present invention are different from those of DE—$A_1$—2 740 280 in the ring system (—C— and —O— at the 3-position). The compounds of the present invention are different from those of GB—A—1 538 240 in the ring system (—C— and —O— at the 4-position).

## Summary of the Invention

In accordance with the present invention, some acyl compounds are prepared of cephalosporin analogs represented by the general formula (I)

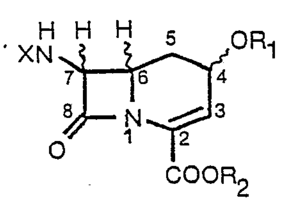

(I)

wherein X represents an acyl group $X_1CO$ used in the chemistry of penicillins and cephalosporins, $R_1$ represents hydrogen or a lower alkyl group, $R_2$ represents hydrogen or a protective group of carboxylic acid and the hydrogens at the 6- and 7-positions have cis configuration and pharmaceutically acceptable salts thereof.

Further, in accordance with the present invention, the optically active acyl compounds are prepared of cephalosporin analogs represented by the general formula (I).

Therapeutically active cephalosporin analogs, particularly, represented by the general formula (II)

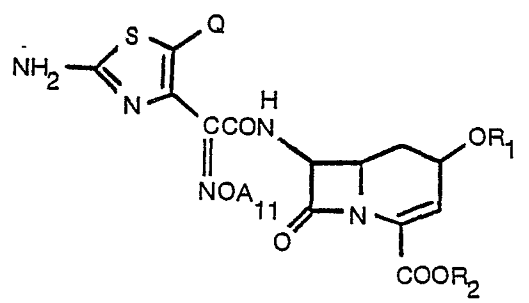

(II)

(wherein Q, $R_1$, $R_2$ and $A_{11}$ have the same significance as defined above) have extremely strong antibacterial activities.

Starting compounds used in the present invention, that is, the compounds represented by the general formula (V)

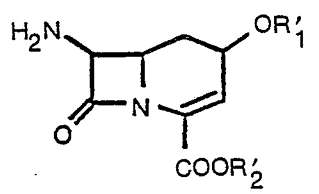

(V)

[wherein $R_1'$ is the same or different from $R_1$ and represents hydrogen, a lower alkyl group having 1 to 5 carbon atoms or a protecting group of the hydroxy group and $R_2'$ is the same or different from $R_2$ and represents hydrogen or a protecting group of carboxy group] are disclosed in DE—A—2911786, and are present as a mixture of equal amounts of optical isomers, Compound (V-1) and Compound (V-2) which are mirror images of each other.

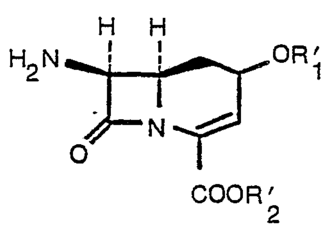

(V–1)

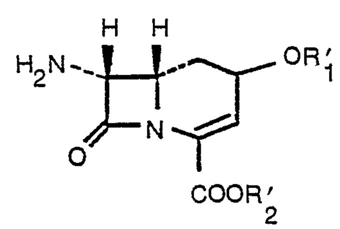

(V–2)

The optically active compounds presented by the present inventors are similarly assumed to have the absolute structure represented by the general formula (V-1) defined above from various properties, stronger antimicrobial activity of the acyl compounds compared with that of the corresponding optically inactive acyl dl-compound, the relationship between the absolute structure of cephalosporins and activities thereof and the data given in Japanese Patent Application Nos. 14533/79 and 14534/79 (published E.P.A., see above).

Similarly, acyl compounds of the optically active cephalosporin analogs presented by the present inventors are assumed to have the absolute structure represented by the general formula (VI)

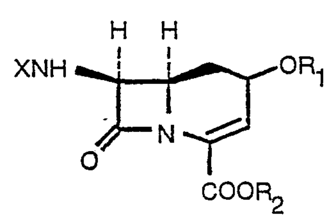

(VI)

In the following, the optically active compounds are described as having the absolute configuration of 7$\alpha$-H and 6$\alpha$-H (6R 7S), i.e. the configuration illustrated by the general formula (V-1) or (VI) and in the following Examples and Reference Examples, the compounds are named according to the assumed absolute structural formula as in Japanese Patent Application Nos. 14533/79 and 14534/79 (pulished E.P.A. see above).

Detailed Description of the Invention

The compound (I) are produced by acylating the compound (V) or the compound (V-1), salts thereof, a derivative wherein the hydrogen in the amino group at the 7-position or the hydroxy group at the 4-position is substituted by a trialkylsilyl group, etc., or a compound functionally equivalent thereto (these are referred to as "7-amino compound" hereinafter) with a carboxylic acid represented by the general formula (VII)

$$X_2COOH \qquad (VII)$$

(wherein $X_2$ represents a group $X_1CO$ or a derivative thereof wherein functional group(s) are protected and $X_1CO$ has the same significance as defined below) or a reactive derivative thereof, and if necessary, eliminating the protective group in the group $X_2$, the protective group $R_2'$ or the protective group of the hydroxy group at the 4-position in a conventional manner.

As the functional groups requiring protection in $X_1$, a hydroxy group, a thiol group, an amino group, a carboxy group and a sulfo group are exemplified. As the protective groups, those employed in the chemistry of peptide synthesis are used. Examples of the protective groups are described in "Protective Groups in Organic Chemistry" J. F. W. McOmie, Plenum Press, 1973.

As the reactive derivatives of the carboxylic acid represented by the general formula (VII), 1) an acid halide, 2) an acid anhydride, 3) a mixed acid anhydride, 4) an active ester, 5) an acid azide are exemplified.

In the foregoing general formula (I), the lower alkyl group $R_1$ is a straight-chain or branched alkyl group having 1 to 5 carbon atoms such as methyl, ethyl, n-propyl, i-propyl, n-butyl, i-butyl, sec-butyl, t-butyl, and the like.

In the general formula (I), the group represented by $R_2$ is a hydrogen or a protective group of carboxylic acid conventionally used in the chemistry of penicillins and cephalosporins, for example, an alkyl group having 1 to 5 carbon atoms such as methyl, ethyl, n-propyl, isopropyl, n-butyl, isobutyl, sec-butyl, t-butyl, a halogenated alkyl group having 1 to 5 carbon atoms such as 2,2,2-trifluoroethyl, 2,2,2-trichloroethyl, an arylmethyl group having 7 to 20 carbon atoms such as benzyl, diphenylmethyl, triphenylmethyl, an arylmethyl group having 7 to 20 carbon atoms and having methoxy, nitro, on the phenyl ring, a substituted silyl group such as trimethylsilyl or triphenylsilyl or a group enzymatically or nonenzymatically readily eliminable *in vivo*, for example, a group represented by the general formula

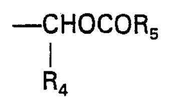

wherein $R_4$ represents a hydrogen atom or a lower alkyl group having 1 to 6 carbon atoms and $R_5$ represents a lower alkyl group having 1 to 6 carbon atoms, a lower alkoxy group having 1 to 6 carbon atoms or a phenyl group, etc.

The acyl group X in the general formula (I) is represented by $X_1CO$ wherein $X_1$ represents a group selected from the following groups:

1) a group represented by the general formula:

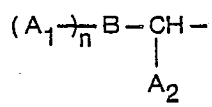

[wherein B represents a cyclohexenyl group, a cyclohexadienyl group, a phenyl group, or a five- or six-membered heterocycle such as furyl, pyrrolyl, thiazolyl, oxazolyl, isothiazolyl, isoxazolyl, imidazolyl, pyrazolyl, triazolyl, tetrazolyl, pyridinyl, pyrimidinyl, pyrazinyl, pyridazinyl, triazinyl, 1,2,4-thiadiazolyl, 5,6-dihydro-1,4-dithiin-2-yl, $A_1$ represents substituent(s) on the ring B, which is selected from a hydrogen atom, a hydroxy group, an alkoxy group having 1 to 4 carbon atoms, a halo group, a nitro group, an amino group, an aminomethyl group, a methylsulfonamido group and an acyloxy group having 1 to 4 carbon atoms, n is 0 or an integer or 1 to 5, and $A_2$ represents a hydrogen atom, an amino group, a hydroxy group, a carboxy group or a sulfo group],

2) a group represented by the general formula:

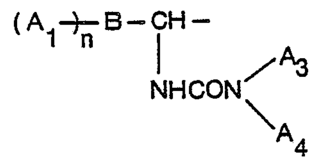

[wherein $A_1$, B and n have the same significance as defined above, $A_3$ and $A_4$ are the same or different and represent a hydrogen atom, an alkyl group having 1 to 4 carbon atoms, a group represented by the general formula

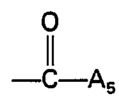

(wherein $A_5$ represents an alkyl group having 1 to 4 carbon atoms) or a group represented by the general formula

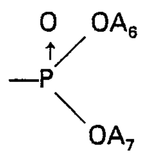

(wherein $A_6$ and $A_7$ are the same or different and represent a hydrogen atom, an alkyl group having 1 to 4 carbon atoms or an alkali metal) and

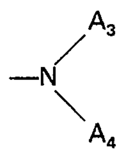

represents a group represented by the general formula

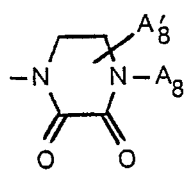

(wherein $A_8$ and $A_8'$ are the same or different and represent a hydrogen atom or an alkyl group having 1 to 4 carbon atoms) or a group represented by the general formula

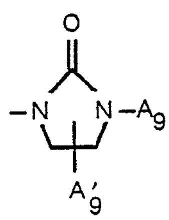

(wherein $A_9$ represents a hydrogen atom, an alkyl group having 1 to 4 carbon atoms, a methanesulfonyl group or a furfurylideneimino group, and $A_9'$ represents a hydrogen atom or an alkyl group having 1 to 4 carbon atoms)], and

3) a group represented by the general formula

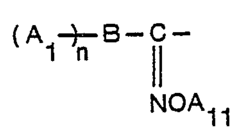

[wherein $A_1$, B and n have the same significance as defined above and $A_{11}$ represents a hydrogen atom, a lower alkyl group having 1 to 6 carbon atoms, a lower alkenyl group having 2 to 6 carbon atoms, a lower alkinyl group having 2 to 6 carbon atoms, a cycloalkyl group having 3 to 6 carbon atoms or an

aryl group and those groups may be substituted by a suitable substituent such as carboxy, cyano, halo, carbamoyl or a lower alkyloxycarbonyl group having 1 to 4 carbon atoms].

In the foregoing, $X_2$ represents a group selected from the following groups:

1') a group represented by the general formula:

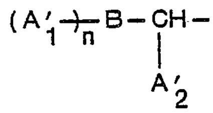

[wherein B and n have the same significance as defined above, $A_1'$ represents a substituent which is selected from a hydrogen atom, a hydroxy group, a protected hydroxy group, an alkoxy group having 1 to 4 carbon atoms, a halo group, a nitro group, a protected amino group, a protected aminomethyl group, a methylsulfonamido group and an acyloxy group having 1 to 4 carbon atoms, and $A_2'$ represents a hydrogen atom, a protected amino group, a hydroxy group, a protected hydroxy group, a carboxy group, a protected carboxy group, a sulfo group or a protected sulfo group],

2') a group represented by the general formula:

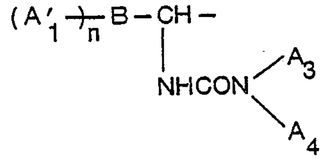

[wherein $A_1'$, $A_3$, $A_4$, B and n have the same significance as defined above], and 3') a group represented by the general formula:

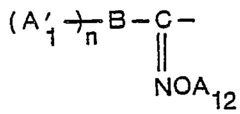

[wherein $A_1'$, B and n have the same significance as defined above and $A_{12}$ represents a hydrogen atom, a lower alkyl group having 1 to 6 carbon atoms, a lower alkenyl group having 2 to 6 carbon atoms, a lower alkinyl group having 2 to 6 carbon atoms, a cycloalkyl group having 3 to 6 carbon atoms or an aryl group and those groups may be substituted with a suitable substituent which is selected from a protected carboxy group, a cyano group, a halo group, a carbamoyl group and a lower alkyloxycarbonyl group having 1 to 4 carbon atoms].

In case that $R_2$ is a hydrogen atom or that X is an acyl group having a carboxy group or a sulfo group, the compounds represented by the general formula (I) may be salts with pharmaceutically acceptable, inorganic or organic bases. Further, in case that X is an acyl group having an amino group, the compounds represented by the general formula (I) may be salts with pharmaceutically acceptable, inorganic or organic acids. As the salts, hydrochlorides, sulfates, phosphates, formates, malates, fumarates, sodium salts, potassium salts, magnesium salts, organic amine salts are exemplified.

The compounds represented by the general formula (I) include all stereoisomers at the 4-, 6- and 7-positions. The compounds represented by the general formula (V) similarly include all stereoisomers at the 4-, 6- and 7-positions. Among the stereoisomers, those which have cis configuration at the 6- and 7-positions have higher antibacterial activities than trans-isomers and therefore the cis-isomers are more useful as antibiotics.

The $=NOA_{11}$ group in the acyl group has the two geometrical isomers, i.e. syn and anti. Since the syn-isomer is superior to the anti-isomer in antibacterial activity, the syn-isomer is more useful as antibiotics.

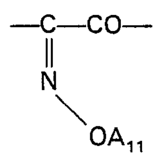

syn isomer

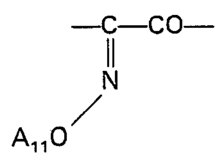

anti isomer

In the present specification, a heterocycle such as 2-amino-thiazolyl group in the acyl group includes its tautomer, 2-imino-thiazolinyl group

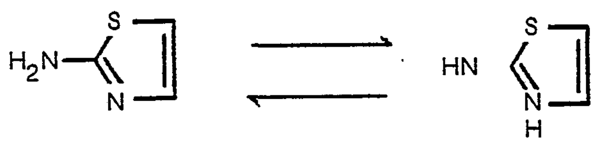

2-amino-thiazolyl group 2-imino-thiazolinyl group

Compound [I] are produced by acylating the compound [V] with a carboxylic acid represented by the general formula (VII) $X_2COOH$ (VII) (wherein $X_2$ has the same significance as defined above).

The acylation reaction is carried out according to a conventional method employed in the chemistry of penicillins and cephalosporins.

Though such methods are described in DE—A—2911787 and GB—A—2017103, the acylation methods are disclosed in the following paragraph.

The compound [V], salts thereof, a derivative wherein the hydrogen in the amino group or the hydroxy group at the 4-position is substituted by a trialkylsilyl group, etc., or a compound functionally equivalent thereto (these are referred to as "7-amino compound" hereinafter) is acylated with a carboxylic acid represented by the general formula (VII) or a reactive derivative thereof, if necessary, followed by elimination of the protective group in the group $X_2$, the protective group $R_2$ or the protective group in the hydroxy group at the 4-position in a conventional manner.

Acylation reactions using the above derivatives are explained in the following.

1) Method using an acid halide

A 7-amino compound and an acid halide are subjected to condensation reaction, preferably in the presence of a proton acceptor.

As the proton acceptor, inorganic bases such as sodium carbonate, potassium carbonate, sodium hydroxide, potassium hydroxide and organic bases such as triethylamine, N-methylmorpholine or pyridine are used.

As the solvent, any inactive solvent which does not affect the reaction, preferably an ether such as tetrahydrofuran, dimethoxyethane, an ester such as ethyl acetate, a halogenated hydrocarbon such as methylenechloride, chloroform, an amide such as dimethylformamide, dimethylacetamide, hexamethylphosphoamide, a sulfoxide such as dimethylsulfoxide or water is used solely or in combination.

The reaction is carried out at a temperature of −20 to 40°C.

2) Method using an acid anhydride

A 7-amino compound and an acid anhydride are subjected to condensation reaction in an inactive solvent.

The solvents, temperature, etc. employed in Method 1) are applied in this method.

The acid anhydride method using dicyclohexylcarbodiimide is preferably used.

3) Method using a mixed acid anhydride

A 7-amino compound and a mixed acid anhydride are subjected to condensation reaction in an inactive solvent.

The mixed acid anhydride is prepared in a conventional manner, for example, by the method wherein a corresponding carboxylic acid, $X_2COOH$ and a chlorocarbonic ester are reacted in the presence of a base.

The solvent, temperature, etc. employed in Method 2) are applied in this method.

4) Method using an active ester

A 7-amino compound and an active ester are subjected to condensation reaction in an inactive solvent.

As the active ester, phenyl ester, p-nitrophenyl ester, p-nitrothiophenyl ester, trichlorophenyl ester, cyanomethyl ester, N-hydroxysuccinimide ester, etc. are used.

The solvents, temperature, etc. employed in the above methods are also applied in this method.

5) Method using an acid azide

A 7-amino compound and an acid azide compound are subjected to condensation reaction according to the above method.

The acid azide may be prepared by reacting the hydrazide of a corresponding carboxylic acid with nitrous acid at a temperature of −20 to 0°C.

In case that the carboxylic acids or reactive derivatives thereof have such functional groups as amino, hydroxy, thiol, carboxy, sulfo, etc. which are susceptible to acylation, those groups are preferably

protected with a suitable protective group prior to the condensation reaction.

As the amino-protecting group, t-butyloxycarbonyl, benzyloxycarbonyl, trichloroethyloxycarbonyl, trityl, formyl, chloroacetyl, trialkylsilyl, proton, β-diketon, β-ketoester, etc. are preferably exemplified. As a compound protected with a proton, the compound represented by the formula:

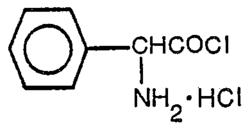

is exemplified. As a compound protected with a β-ketoester, the compound represented by the formula:

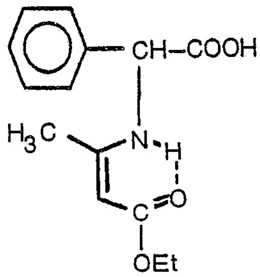

is exemplified.

As the hydroxy-protecting group, benzyl, benzyloxycarbonyl, trityl, tetrahydropyranyl or t-butyl are exemplified.

As the carboxy-protecting group, t-butyl, benzyl, p-methoxybenzyl, p-nitrobenzyl and benzhydryl are mentioned.

In the acylation reaction, the protection of the hydroxy group at the 4-position in Compound [V] is not essential. When the hydroxy group is protected, the protective group is eliminated after the acylation. As the protective group of the hydroxy group, a trialkylsilylated compound which is used in the protection of the amino group is employed.

As the thiol-protecting group, benzyl, trityl, benzyloxycarbonyl and p-nitrobenzyl are mentioned.

Introduction and elimination of the above protecting groups are carried out by a conventional method described in "Protective Group in Organic Chemistry".

Elimination of the carboxy-protective groups ($R_2'$) is carried out according to a conventional method employed in the field of the synthetic chemistry of penicillins and cephalosporins, preferably, 1) catalytic reduction, 2) acidolysis, 3) cleavage reaction using a Lewis acid, 4) hydrolysis, 5) reduction other than catalytic reduction using Zn-acetic acid and 6) a method using an esterase.

The optically inactive compounds of the starting compounds used in the present invention are prepared according to the method disclosed in Japanese Published Unexamined Patent Application Nos. 128591/79, 4337/80 and 49376/80 and Japanese Patent Application No. 8408/79 DE—A—2911787, GB—A—2017103), an example of which is illustrated in the following Reference Flow Sheet [I].

REFERENCE FLOW SHEET [I]

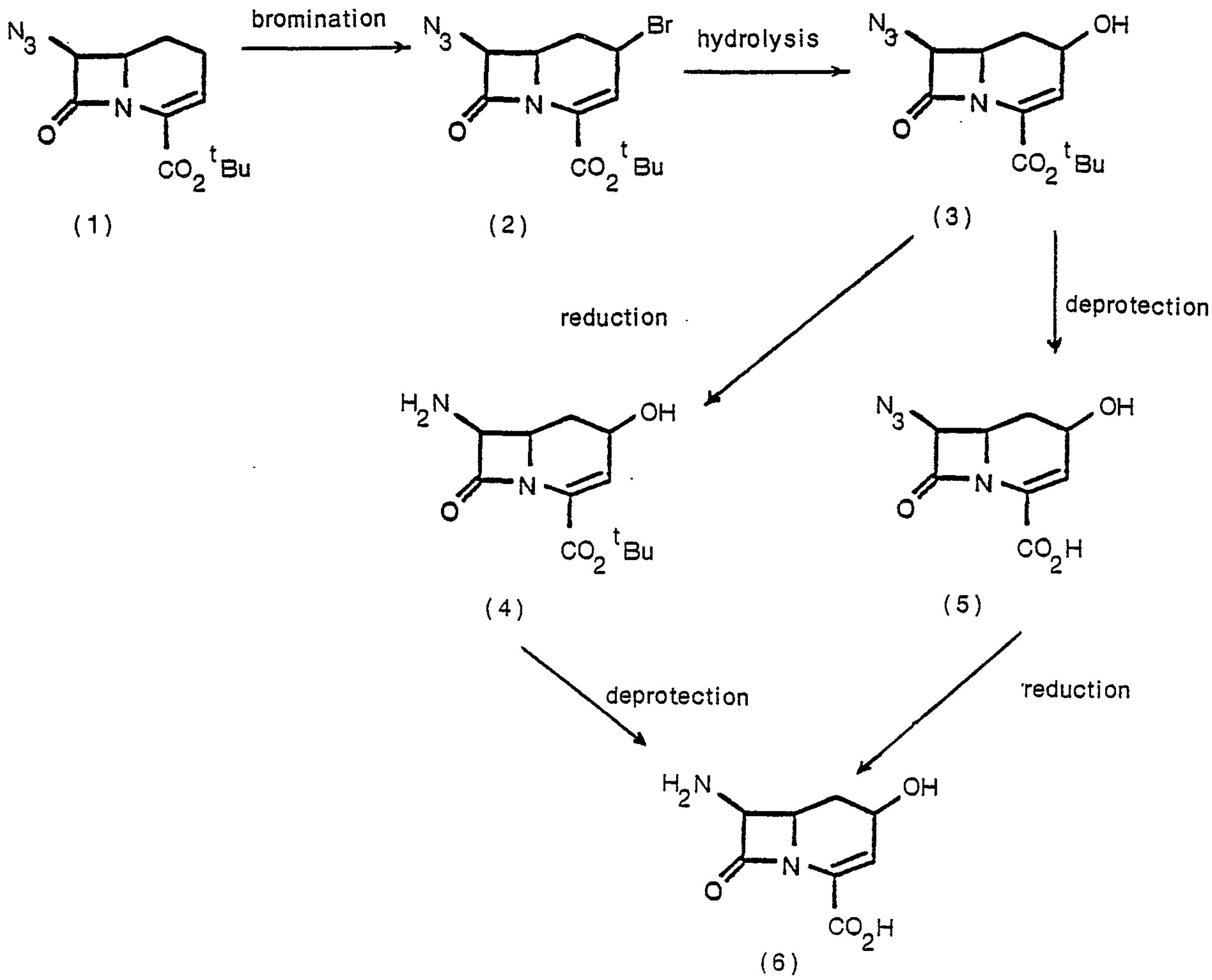


(1) (2) (3) (4) (5) (6)

In this method, compounds wherein the hydroxy group at the 4-position has cis configuration to the hydrogens at the 6- and 7-positions are usually produced. The compounds wherein the hydroxy group has trans configuration to the hydrogens at the 6- and 7-positions are prepared according to the process in the following Reference Flow Sheet [II].

REFERENCE FLOW SHEET [II]

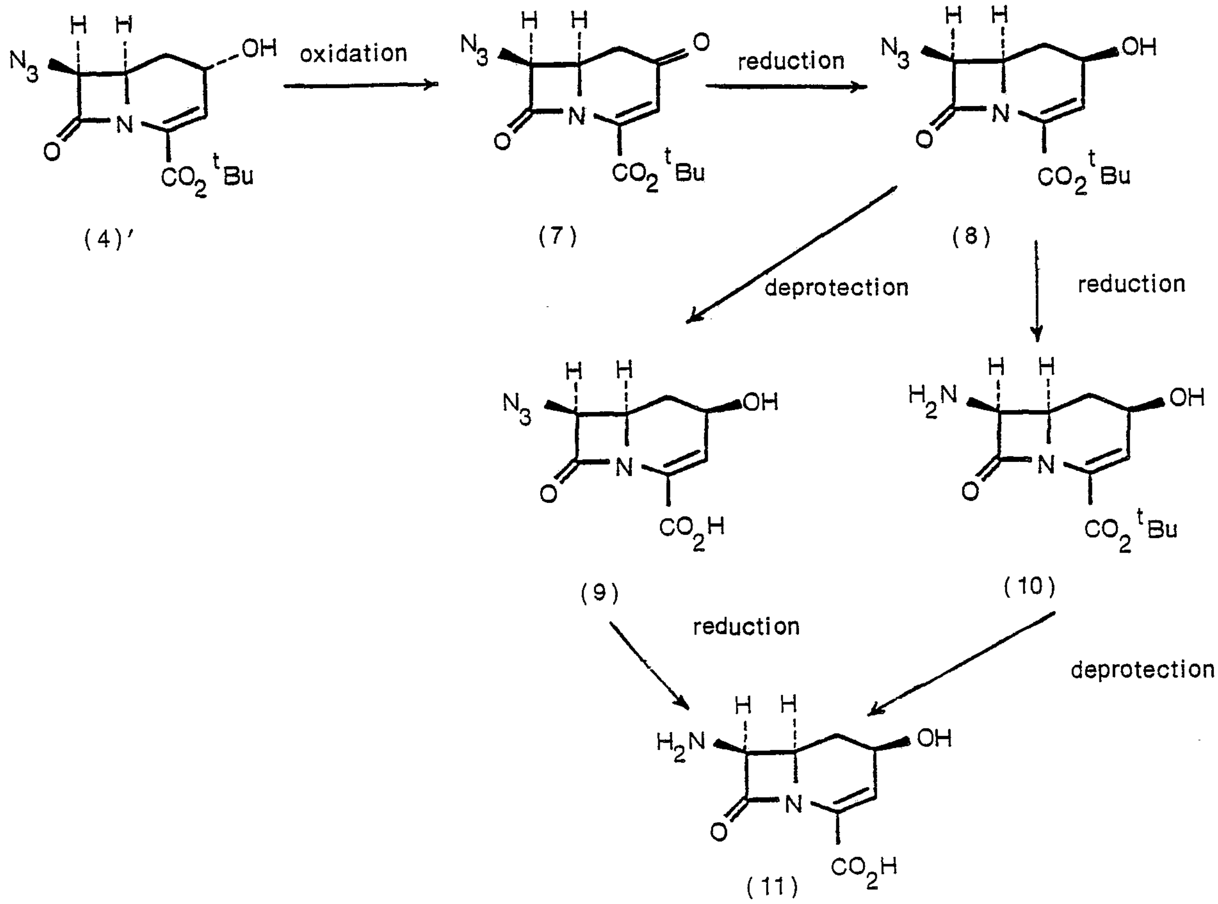


(4)' (7) (8) (9) (10) (11)

Compounds (4), (6), (10) and (11) are used as the starting compound, Compound (V). Compound (1) in the Reference Flow Sheet [I] is produced according to the method disclosed in Japanese Published Unexamined Patent Application No. 128591/79, more preferably, Japanese Patent Application No. 53076/79, examples of which are illustrated in the following Reference Flow Sheet [III] and Reference Flow Sheet [IV].

REFERENCE FLOW SHEET [III]

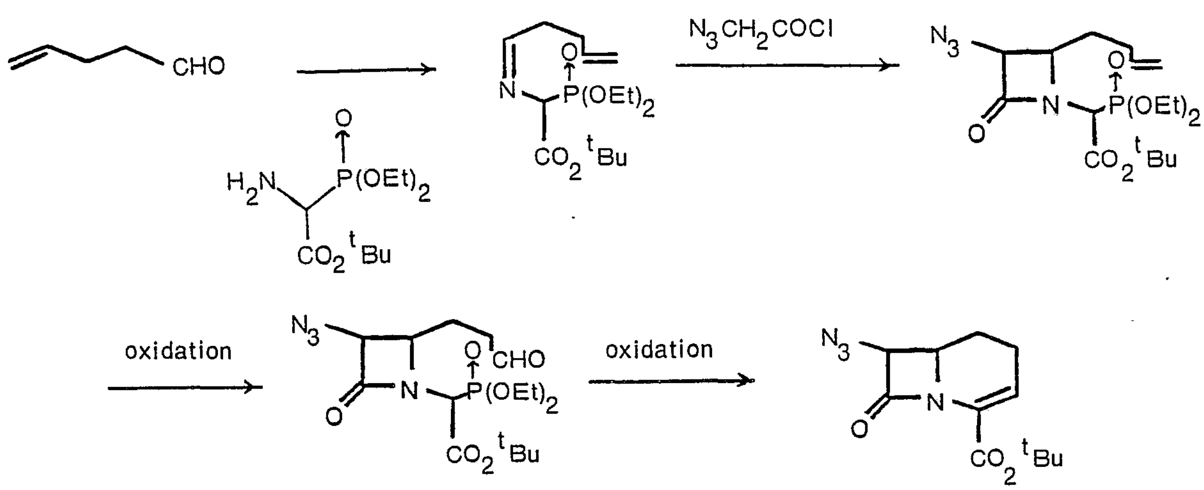

REFERENCE FLOW SHEET [IV]

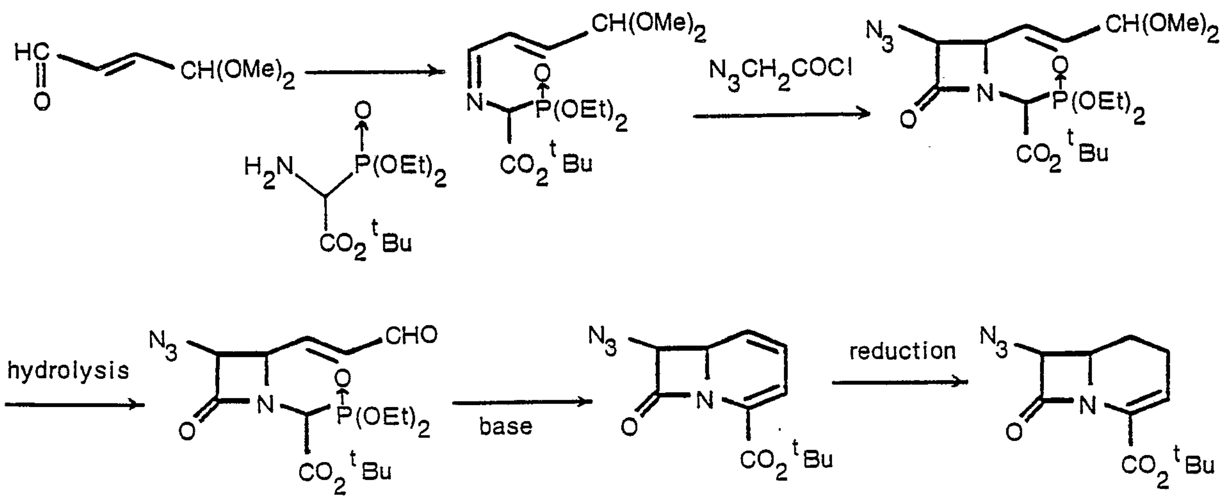


The methods in Reference Flow sheets [I], [II], [III] and [IV] described above are explained in detail in Reference Examples 1 to 9 below.

Further, the optically active compounds of the starting compounds used in the present invention have been first prepared according to a similar method disclosed in Japanese Patent Application Nos. 14533/79 and 107070/79 (European Patent Publication No. 14475) by the inventors.

Optically active compounds of the cephalosporin analogs represented by the general formula (V) or compounds represented by the assumed absolute structural formula (V-1) are produced by optically selective deacylation of the compound represented by the general formula (VIII)

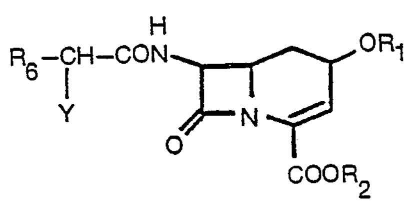
(VIII)

wherein $R_6$ represents a substituted or unsubstituted unsaturated six-membered carbocycle, five- or six-membered heterocycle or a phenoxy group, Y represents a hydrogen, an amino group, a hydroxy group or a lower alkyl group, and $R_1$ and $R_2$ have the same significance as defined above.

As the unsaturated six-membered carbocycle and five- or six-membered heterocycle, a phenyl group, a cyclohexenyl group, a cyclohexadienyl group, a thienyl group, a furyl group, a pyrrolyl group, a thiazolyl group, an isothiazolyl group, an oxazolyl group, an iso-oxazolyl group, an imidazolyl group, a pyrazolyl group, a triazolyl group, a tetrazolyl group, a pyridinyl group, and a pyrazinyl group are exemplified. As the substituent, a hydroxy group, a halo group, a nitro group, a methansulfonamide group, and the like are mentioned. As the lower alkyl group, straight-chain or branched alkyl groups having 1 to 5 carbon atoms such as methyl, ethyl, n-propyl, i-propyl, n-butyl, i-butyl, sec-butyl, t-butyl are mentioned.

The optically selective deacylation of Compound [VIII] to optically active Compound [V-1] is carried out in the presence of cells or treated matter of culture broth of a microorganism capable of producing optically active Compound [V-1] by optically selective deacylation of Compound [VIII] or an enzyme obtained from the microorganisms.

As the microorganism having the ability of optically selective deacylation, microorganisms belonging to the genus *Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococcus, Spirillum, Bacillus, Pseudomonas, Flavobacterium, Brevibacterium, Protaminobacter, Proteus, Beneckea, Micrococcus, Mycoplana, Rhodopseudomonas, Nocardia, Neurospora, Talaromyces* or *Acinetobacter* are used.

The following strains are examples of the microorganism.

| | | |
|---|---|---|
| 1. | *Aeromonas hydrophila* | IFO 12634 |
| 2. | *Achromobacter aceris* | IFO 3320 |
| 3. | *Arthrobacter simplex* | ATCC 15799 |
| 4. | *Acetobacter aurantius* | IFO 3245 |
| 5. | *Acetobacter sp.* | ATCC 21760 |
| 6. | *Alcaligenes faecalis* | ATCC 8750 |
| 7. | *Escherichia coli* | ATCC 11105 |
| 8. | *Escherichia coli* | ATCC 13281 |
| 9. | *Xanthomonas citri* | IFO 3835 |
| 10. | *Xanthomonas physalidicola* | IFO 13555 |
| 11. | *Kluyvera citrophila* | ATCC 21285 |
| 12. | *Gluconobacter liquefaciens* | ATCC 14835 |
| 13. | *Gluconobacter deoxyacetonicus* | IFO 3271 |
| 14. | *Clostridium acetobutylicum* | ATCC 824 |
| 15 | *Comamonas terrigena* | IFO 12685 |
| 16. | *Corynebacterium tritici* | IFO 12164 |
| 17. | *Sarcina lutea* | ATCC 9341 |
| 18. | *Staphylococcus aureus* | IFO 3060 |
| 19. | *Spirillum methamorphum* | IFO 12012 |
| 20. | *Bacillus megaterium* | ATCC 14945 |
| 21. | *Pseudomonas melanogenum* | ATCC 17808 |
| 22. | *Pseudomonas aeruginosa* | IFO 3451 |
| 23. | *Flavobacterium sp.* | ATCC 21429 |
| 24. | *Brevibacterium cerinum* | ATCC 15112 |
| 25. | *Protaminobacter alboflavus* | IFO 13221 |
| 26. | *Proteus rettgeri* | ATCC 9250 |
| 27. | *Beneckea hyperoptica* | ATCC 15803 |
| 28. | *Micrococcus luteus* | AHU 1427 |
| 29. | *Mycoplana bullata* | IFO 13267 |
| 30. | *Mycoplana dimorpha* | IFO 13213 |
| 31. | *Rhodopseudomonas spheroides* | ATCC 21286 |
| 32. | *Nocardia globerula* | ATCC 21022 |

| | | |
|---|---|---|
| 33. | *Neurospora sitophila* | IFO 4596 |
| 34. | *Talaromyces luteus* | IFO 6896 |
| 35. | *Acinetobacter calcoaceticus* | ATCC 21288 |

The microorganism Nos. 32 to 25 are used in the preparation of Compound [VIII] wherein $R_6$ is a phenoxy group and Y is a hydrogen.

In the optical selective deacylation, the following substances may be used instead of the enzyme produced by the microorganism mentioned above.

1. Culture liquor of the microorganism or treated matter thereof.

2. Cell bodies recovered from the culture broth by centrifugation which may be further washed with saline water (usually about 1%), buffer solution and the like, or a cell suspension.

3. Distrupted cell suspension, i.e., a suspension of the cell bodies disrupted mechanically or chemically.

4. Cell free extract, i.e., a liquid obtained by removing the disrupted cell bodies from the disrupted cell suspension.

5. Purified enzyme solution which is obtained by recovering the enzyme protein with ammonium sulfate from the cell free extract and subjecting the enzyme protein to gel filtration, ion-exchange cellulose column chromatography or ion-exchange Sephadex column chromatography.

Cells or the purified enzyme immobilized by a conventional method may be used.

The reaction is carried out at a temperature of 0 to 50°C, preferably 20 to 45°C and at a pH of 4 to 10 in an inactive solvent which does not affect the reaction.

As the solvent, water is most preferably used. In order to dissolve the substrate(s), cephalosporin analog(s), organic solvents such as acetone, methanol, ethanol, N,N-dimethylformamide, dimethylsulfoxide may be used. It is effective to add phosphate buffer, Veronal buffer of citric acid buffer to control the pH in the reaction. Reaction time which is influenced by the kind and concentration of enzymes, the kind and concentration of substrates, reaction temperature or reaction pH, is generally 30 minutes to 24 hours. It is most preferable to terminate the reaction when the reaction ratio reaches maximum.

The concentration of cells is preferably 1 to 50 mg by dry weight per 1 ml of the reaction solution. When a purified enzyme is used, it is appropriate to use the amount of the enzyme having the same activity as that of the dry cell. The substrate Compound [VIII] is used in an amount of 0.5 to 50 mg per 1 ml of the reaction solution.

When enzymes preventing the reaction such as $\beta$-lactamase or esterase are contained in the cell body, a mutant strain having a reduced productivity of the enzyme may be used. Further, inhibitors against such enzymes may be added in the reaction system to raise the reaction ratio.

After the completion of the reaction, isolation of the desired compound is carried out by a conventional method employed in the isolation and purification of organic compounds from culture liquors such as adsorption using various carriers, ion-exchange chromatography, gell filtration or liquid-liquid extraction.

Among the compounds represented by the general formula (V), the optically active compounds of the cephalosporin analogs represented by the general formula (V-3)

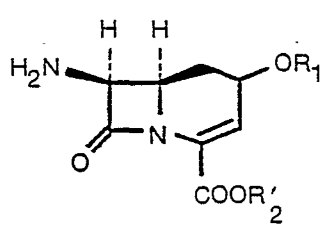

(V–3)

(wherein $R_1$ has the same significance as defined above, $R_2'$ represents a protective group of carboxylic acid and the hydrogens at the 6- and 7-positions have cis configuration) are obtained by the esterification of the optically active cephalosporin analogs represented by the general formula (V-4)

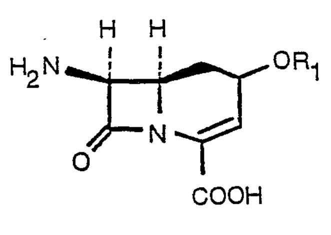

(V–4)

(wherein $R_1$ has the same significance as defined above, and the hydrogens at the 6- and 7-positions have cis configuration) by a conventional method.

The compound [VIII] is produced by acylating Compound [V], a derivative of Compound [V] wherein the amino group at the 7-position or the hydroxy group at the 4-position is suitably protected, if necessary, or a functionally equivalent compound thereof with a carboxylic acid represented by the general formula (IX)

$$R_6—CH(Y)—COOH \quad (IX)$$

wherein $R_6$ and Y have the same significance as defined above, a reactive derivative of the carboxylic acid or a derivative of the carboxylic acid wherein amino group or hydroxy group is protected with a protective group, and thereafter eliminating the protective group by a conventional method, if necessary. Those methods for producing compounds [VIII] are described in co-pending application EP—A—0027882.

The protective groups, elimination methods and the acylation methods mentioned above are employed in the production of compound [VIII] and those are shown in Reference Example.

The present invention is explained by the following Examples.

## Example 1

Preparation of (4S, 6R, 7S)-7-amino-4-hydroxy-1-azabicyclo[4,2,0]oct-2-en-8-on-2-carboxylic acid:

1-1. Preparation of disrupted cell suspension

1) Cultivation of a microorganism having an ability of optically selective deacylation

As the seed strain, *Kluyvera citrophila* ATCC 21285 [Biological properties are described in J. General Applied Microbiology *3*, 28—31 (1957)] is used.

As the seed medium, an aqueous solution containing 1% polypeptone, 1% yeast extract, 0.5% meat extract, 0.5% sodium glutamate and 0.25% sodium chloride and adjusted at a pH of 7.0 with 5N—NaOH is used. One loopful of the seed strain is inoculated into 10 ml of the seed medium in a large test tube (50 ml) and culturing is carried out at a temperature of 30°C for 24 hours. The whole of the seed broth is inoculated into 300 ml of the culture medium in 2 l of an Erlenmeyer flask and culturing is carried out with shaking at a temperature of 30°C. The composition of the culture medium is the same as that of the seed medium.

2) Preparation of disrupted cell suspension.

After culturing for 24 hours, the culture broth is subjected to centrifugation to obtain cell bodies. The cells are washed twice with 50 ml of 0.9% saline solution and suspended in a concentration of 40 mg/ml by dry weight in 1/30 M phosphate buffer solution (pH 8.0).

1-2. Preparation of a substrate solution

In this step, 200 mg of (±)-cis-7β-phenylacetamido-4a-hydroxy-1-azabicyclo[4,2,0]oct-2-en-8-on-2-carboxylic acid obtained as in Reference Example 9 below is added into 9 ml of 1/30 M phosphate buffer (pH 8.0). Since the compound is not dissolved, 2N—NaOH is added in a small portion and the mixture is again adjusted at a pH of 8.0 to dissolve the compound. Finally, deionized water is added to make 10 ml of a solution.

1-3. Enzyme reaction

In this step, 10 ml of the disrupted cell suspension mentioned above is added in 10 ml of the substrate solution and enzyme reaction is carried out at a temperature of 40°C for 80 minutes. Time course of the reaction is illustrated in Table 1.

Table 1

| Reaction period (minutes) | The amount of Compound [I-1]+++ produced (mg/ml) | Yield (Mol ratio, %) |
|---|---|---|
| 10 | 0.8 | 12.5 |
| 20 | 1.3 | 20 |
| 40 | 1.8 | 29 |
| 60 | 2.1 | 33 |
| 80 | 2.2 | 35 |

+++ Compound (I-1) wherein $R_1$ is H and $R_2$ is H.

1-4. Isolation and purification of the desired compound

(a) After the completion of the reaction, cells are removed by centrifugation from the reaction solution. The supernatant is concentrated under reduced pressure and on a column (diameter: 0.88 cm, height; 70 cm) packed with 43 ml of Diaion HP-20AG (100—200 mesh, product of Mitsubishi Kasei Kogyo Co., Ltd.). Elution is carried out with deionized water and the desired compound is eluted from 36 ml of 45 ml of the eluate. The eluate is concentrated under reduced pressure and subjected to high speed liquid chromatography using TRI ROTAR (product of Nippon Bunko Co., Ltd.) and Shodex OH Pak B-804 (product of Showa Denko Co., Ltd.). Elution is carried out with water. Eluates are concentrated under reduced pressure and lyophilized to obtain 37.6 mg of a white powder.

Properties of the product are as follows.

$IR\nu_{max}^{KBr}$ ($cm^{-1}$): 3530, 3190, 1746, 1620, 1550

NMR ($D_2O$ with DSS [sodium-2,2-dimethyl-2-silapentane-5-sulfonate] as an international standard) $\delta$: 6.17 (1H, d, J=5.4Hz), 4.95 (1H, d, J=5.4Hz), 4.59 (1H, m), 4.11 (1H, m), 2.23 (1H, m), 1.80 (1H, m)

The properties of the product coincide well with those of the corresponding dl-compound. The value of optical rotation is $[\alpha]_D^{30°} = +24.2°$ (C = 0.153, $H_2O$).

It is assumed that the product is a mixture of the desired compound obtained by purifying as in Methods b) and c) and a trace amount of a dextrorotatory compound.

The compound shows a ninhydrin positive single spot at an Rf value of 0.38 on silica gel thin layer chromatography [thin layer plate Merck Art 5721 (product of E. Merck & Co.], solvent for development, ethanol:acetic acid:water = 4:1:1]. The Rf value coincides with that of the optically inactive dl-compound.

(b) After the completion of the enzyme reaction carried out as in Example 1-1 to 1-3, cell bodies are removed from the reaction solution by centrifugation and the supernatant is concentrated under reduced pressure. The concentrate is charged on a column (diameter: 0.88 cm, height: 70 cm) packed with 43 ml of Diaion HP-20AG (product of Mitsubishi Kasei Kogyo Co., Ltd., 100—200 mesh). Elution is carried out with water. Eluted fractions (36 ml to 45 ml) containing the desired compound are again concentrated under reduced pressure. The concentrate is charged on a column (diameter: 0.88 cm, height: 33 cm) packed with 20 ml of Diaion WA—30—S (product of Mitsubishi Kasei Kogyo Co., Ltd.) which is in advance made acetic acid form by passing 40 ml of 0.5 N aqueous acetic acid through the column. After 20 ml of water is passed through the column to eliminate contaminants such as inorganic ions, 0.5 N aqueous acetic acid is passed through it. The desired product is eluted in the fractions (30 to 45 ml) of 0.5 N aqueous acetic acid. The fractions are lyophilized to obtain 32 mg of the desired product as pale yellow powder. Properties of the product are as follows and the product is identified as the acetate of the desired compound.

$IR\nu_{max}^{KBr}$ ($cm^{-1}$): 3400, 1805, 1760, 1745 (sh), 1600, 1560 (sh), 1410

NMR ($D_2O$, with DSS as an internal standard) $\delta$ (ppm): 6.16 (1H, d, J=5.4Hz), 4.89 (1H, d, J=5.4Hz), 4.57 (m, superimposed with the signal due to $H_2O$), 4.08 (1H, m), 2.21 (1H, m), 1.97 (3H, s), 1.78 (1H, m)

Optical rotation $[\alpha]_D^{20°} = -62.1°$ (c = 0.16, 1M phosphate buffer, pH 7.0)

(c) Preparation of (4S, 6R, 7S)-7-amino-4-hydroxy-1-azabicyclo[4,2,0]oct-2-en-8-on-2-carboxylic acid (Alternative method):

(c-1) Preparation of (4S, 6R, 7S)-7-t-butoxycarbonylamino-4-hydroxy-1-azabicyclo[4,2,0]oct-2-en-8-on-2-carboxylic acid:

After the completion of the enzyme reaction carried out as in Example 1-1 to 1-3, cell bodies are removed from the reaction solution by centrifugation and the supernatant is concentrated under reduced pressure. The concentrate is charged on a column (diameter: 0.88 cm, height; 70 cm) packed with 43 ml of Diaion HP-20 (product of Mitsubishi Kasei Kogyo Co., Ltd., 100—200 mesh). Elution is carried out with water. Eluted fractions (36 ml to 45 ml) are combined, concentrated under reduced pressure and lyophilized to obtain 100 mg of a white powder. To the powder are added 1.0 ml of dioxane, 1.0 ml of water, 21 $\mu l$ of triethylamine and 40 mg of S-t-butoxycarbonyl-4,6-dimethyl-2-mercaptopyridine and the mixture is stirred at room temperature for 4 days and at 40°C for 17 hours. The reaction solution is concentrated under reduced pressure to reduce the volume of about half. The residue is washed with ethyl acetate three times and the pH of the water layer is adjusted to about 3 with 10% aqueous citric acid under ice cooling. After extracting with ethyl acetate five times, the ethyl acetate layer is washed with saturated saline solution twice, dried with anhydrous sodium sulfate and concentrated under reduced pressure to obtain 6.5 mg of white crystals. The product is identified as the desired compound based on the following properties.

$IR\nu_{max}^{KBr}$ ($cm^{-1}$): 3450, 3350, 1785, 1765, 1640, 1540

NMR ($CD_3OD$) $\delta$ (ppm): 6.42 (1H, d, J=5.4Hz), 5.27 (1H, d, J=5.4Hz), 4.41 (1H, m), 3.95 (1H, m), 2.3—1.2 (2H, m), 1.46 (9H, s)

Optical rotation $[\alpha]_D^{21°} = -38.2°$ (c = 0.11, $CH_3OH$)

(c-2) Preparation of (4S, 6R, 7S)-7-amino-4-hydroxy-1-azabicyclo[4,2,0]oct-2-en-8-on-2-carboxylic acid:

In this process, 3 ml of anhydrous methylene chloride is added to 63 mg of (4S, 6R, 7S)-7-t-butoxycarbonyl-amino-4-hydroxy-1-azabicyclo[4,2,0]oct-2-ene-8-on-2-carboxylic acid obtained as in Example c-1 and 3 ml of trifluoroacetic acid is added with stirring under ice cooling. The mixture is stirred at the same temperature as above for 3.5 hours. Thereafter, the reaction solution is concentrated under reduced pressure to obtain a brown oily product. The product is treated with ethyl ether to obtain 35 mg of a crude desired product as a yellow powder. The product is charged on a column packed with 50 ml of Diaion HP-20AG (product of Mitsubishi Kasei Kogyo Co., Ltd.) and elution is carried out with water. Fractions positive to ninhydrin color reaction are combined and concentrated under reduced pressure to obtain 31 mg (47.0%) of the trifluoroacetate of the desired compound. The product is identified as the trifluoroacetate of the desired compound based on the following properties.

$IR\nu_{max}^{KBr}$ ($cm^{-1}$): 3400, 1800, 1780, 1680, 1620

NMR ($D_2O$) $\delta$ (ppm): 6.31 (1H, d, J=5.4Hz), 5.00 (1H, d, J=5.4Hz), 4.60 (1H, m), 4.16 (1H, m), 2.37—1.66 (2H, m)

Optical rotation $[\alpha]_D^{21°} = -61.9°$ (c = 0.0743, $H_2O$).

The compounds obtained in steps (b) and (c) behave exactly same as the compound in step (a) in thin layer chromatography under the same conditions.

## Example 2

Preparation of (4S,6R,7S)-7-amino-4-hydroxy-1-azabicyclo[4,2,0]oct-2-en-8-on-2-carboxylic acid: (Alternative method).

2-1. Preparation of immobilized enzyme

Cell bodies of *Kluyvera citrophila* ATCC 21285 cultivated in five 2 l flasks as in Example 1-1 are suspended in 1/30 M phosphate buffer (pH 7.0) in a concentration of 40 mg/ml as dry weight. The cells are subjected to ultrasonic disintegration at 200 W for 2 minutes using ultrasonic disintegrator Model UR · 200P (product of Tomy Seiko Co., Ltd.). The disrupted cells are subjected to centrifugation to obtain a supernant. After adding 1% (weight) of the sulfate of streptomycin, the supernatant solution is allowed to stand overnight. Nucleic acid is removed from the solution and ammonium sulfate is added in a concentration of 70% saturation to deposit emzyme proteins. The deposit is recovered by centrifugation and again dissolved in 50 ml of deionized water. The solution is subjected to dialysis for desalting. The enzyme solution is concentrated under reduced pressure to 10 ml and 0.5 ml of 1 M acetic acid — sodium acetate buffer (pH 5) is added. Separately, 10 ml of Diaion WK-10 (product of Mitsubishi Kasei Kogyo Co., Ltd.) is pretreated in 1/20 M acetic acid — sodium acetate buffer (pH 5). The enzyme solution and WK-10 are mixed and the mixture is stirred at a temperature of 30°C overnight. Thus, an immobilized enzyme is prepared.

2-2. Reaction and isolation and purification of the desired compound

The immobilized enzyme (10 ml) mentioned above and 10 ml of a substrate solution prepared as in Example 1-2 are mixed in a large tube and stirred at a temperature of 40°C for 2 hours. Reaction solution is subjected to decantation and purification as in Example 1-4. The product obtained shows almost same properties as those in Example I.

## Example 3

Preparation of (4S,6R,7S)-7-[2-(2-amino-4-thiazolyl)-2-syn-methoxyiminoacetamido]-4-hydroxy-1-azabicyclo[4,2,0]oct-2-en-8-on-2-carboxylic acid:

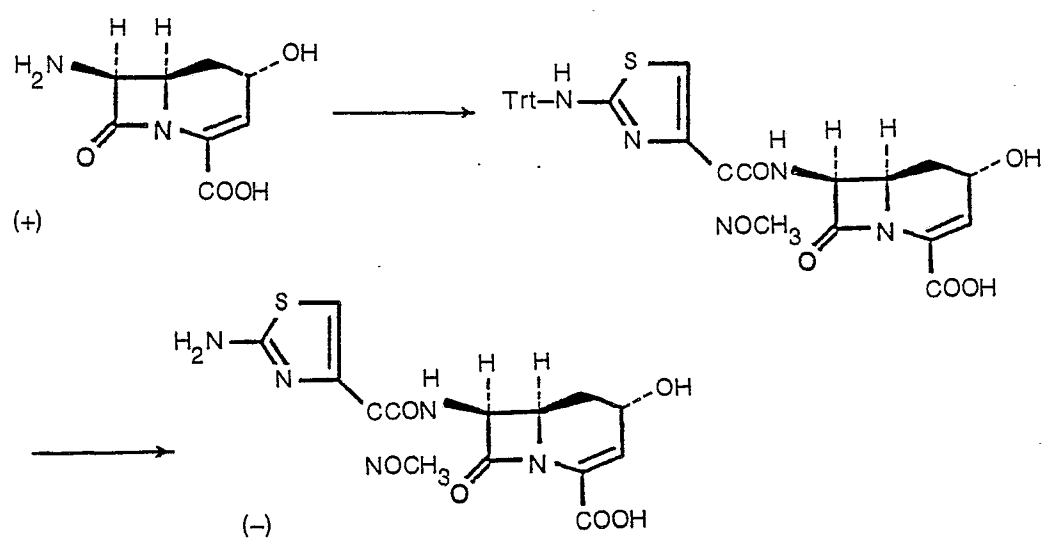
(+)
(–)

In this Example, 78.8 mg (0.178 mmole) of 2-(2-tritylamino-4-thiazolyl)-2-syn-methoxyimino-acetic acid is dissolved in 0.8 ml of anhydrous dichloromethane and 25 $\mu$l (0.178 mmole) of triethylamine is added at a temperature of −20°C. Then, after adding 37 mg (0.178 mmole) of phosphorus pentachloride, the mixture is allowed to react with stirring at a temperature of −20°C for 40 minutes and concentrated under reduced pressure. The residue is dissolved in 2 ml of anhydrous tetrahydrofuran to make an acid chloride solution.

Separately, 34 mg (0.162 mmole) of (4S,6R,7S)-7-amino-4-hydroxy-1-azabicyclo[4,2,0]oct-2-en-8-on-2-carboxylic acid [referred to as Compound (A) hereinafter] obtained as in Example 1-4-a is dissolved in a mixture of 1 ml of tetrahydrofuran and 1 ml of water and 79$\mu$l (0.565 mmole) of triethylamine is added. The acid chloride solution prepared above is added dropwise to the solution with stirring under ice cooling and further 20 $\mu$l of triethylamine is added.

The mixture is allowed to react for two hours and 45 minutes under ice cooling. Then, the mixture is adjusted to a pH of 2.0 with 10% hydrochloiric acid and extracted twice with 10 ml of ethyl acetate. The ethyl acetate layers are washed with 10 ml of saturated saline solution, dried with saturated sodium sulfate and concentrated under reduced pressure to obtain 114 mg of a crude acyl compound. The product is dissolved in 10 ml of 50% acetic acid and stirred at a temperature of 50°C for 30 minutes. The solution is cooled to room temperature and concentrated. The residue is dissolved in 1 ml of methanol. 20 ml of ether and 20 ml of n-hexane are added to the solution and the mixture is subjected to centrifugation to obtain a deposit. The deposit is lyophilized to obtain 51 mg of a solid. The solid is dissolved in a mixture of methanol and water (1:1). The solution is charged on a column packed with 30 ml of HP-20AG and elution is carried out with 40 ml of a mixture of water and methanol (10:1). 30 ml of a mixture of water and methanol (4:1) and 150 ml of a mixture of water and methanol (1:1). Fractions showing an Rf value of 0.3 by silica gel thin layer chromatography [plate: Merck Art. 5719 (product of E. Merck & Co.,), solvent:butanol:acetic acid:water = 4:1:1] are combined and concentrated under reduced pressure. The concentrate is dissolved in 20 ml of ether and 20 ml of n-hexane to obtain a precipitate. The precipitate is recovered by centrifugation and lyophilized to obtain 29.8 mg (yield 45.5%) as white crystals. Properties of the product are as follows.

IR$\nu_{max}^{KBr}$ (cm$^{-1}$): 3450, 1775, 1670, 1635, 1550

NMR (DMSO $d_6$-$CD_3OD$) $\delta$: 9.28 (1H, d, J=8.4Hz), 7.07 (2H, s), 6.75 (1H, s), 6.27 (1H, d, J=5.4Hz), 5.56 (2H, m), 2.77—2.04 (2H, m)

The signal of —$OCH_3$ is superimposed with the signals due to the solvent.

$[\alpha]_D^{24°} = -32°$ (c = 0.5, methanol)

For further purification, 200 mg of the crystals obtained as in the above is dissolved in 1 ml of methanol and 1 ml of hot water is added under heating at 50°C. The solution is allowed to stand at room temperature to obtain white crystals. The crystals obtained by repeating crystallization processes twice is washed with 1 ml of water and dried in vacuo at 45°C for 12 hours. Yields 120 mg. The properties of the products are as follows.

Melting point: The product turns purple at about 140°C and gradually brown and decomposes at 176 to 178°C.

$[a]_D^{19°}$: −1° (c = 0.9, methanol)

NMR (DMSO-$d_6$, 100 M) $\delta$: 1.31 (1H, br), 9.25 (1H, d, J = 8.3Hz), 7.20 (2H, br), 6.76 (1H, s), 6.25 (1H, d, J=5.4Hz), 5.48 (1H, dd, J=8.3, 5.1Hz), 5.26 (1H, br), 4.30 (1H, m), 3.84 (3H, s), 1.43—2.05 (2H, m). The signal of the proton at the 6-position is superimposed with the signal of —$OCH_3$ ($\delta$ 3.84).

Elementary analysis

| | | | |
|---|---|---|---|
| Found | C: 43.33%, | H: 4.43%, | N: 17.89% |
| Calculated as $C_{14}H_{15}N_5O_6S$ 1/2 $H_2O$ | C: 43.08%, | H: 4.13%, | N: 17.94% |

High resolution mass spectrum

The above crystals which are heated at 60°C for 5 hours in N,O-bistrimethylsilylacetamido are provided as the sample.

Mass = 669.23408 ($C_{26}H_{47}O_6N_5SSi_4$)

## Example 4

Preparation of (4S,6R,7S)-7-[2-(2-amino-4-thiazolyl)-2-syn-methoxyiminoacetamido]-4-hydroxy-1-azabicyclo[4,2,0]oct-2-en-8-on-2-carboxylic acid: (Alternative method).

In this example, the procedure of Example 3 is repeated except that the same amount of Compound (A) obtained in Example 1-4-b or 1-4-c is used in place of Compound (A) obtained in Example 1-4-a. The product obtained shows almost same physicochemical properties as those obtained in Example 3.

## Example 5

Antimicrobial activities of the compound obtained in Examples 3, 8 and 12 are as follows: Heart Infusion Agar Dilution Method (pH 7.2) is used. Cefazolin is used as a control.

| Microorganism | MIC (μg/ml) | | | |
|---|---|---|---|---|
| | A* | B* | C* | Cefazolin |
| *Staphylococcus aureus* 209–P | 3.12 | 12.5 | 3.12 | 0.02 |
| *Staphylococcus aureus* Smith | 12.5 | 25 | 25 | 0.78 |
| *Staphylococcus epidermidis* | 25 | 50 | 12.5 | 0.78 |
| *Echerichia coli* NIHJC–2 | 0.02 | 0.05 | 6.25 | 1.56 |
| *Escherichia coli* GN2411–5 | ≤0.01 | 0.02 | 3.12 | 0.78 |
| *Escherichia coli* Juhl | ≤0.01 | 0.05 | 3.12 | 1.56 |
| *Klebsiella pneumoniae* 8045 | ≤0.01 | 0.02 | 3.12 | 1.56 |
| *Klebsiella pneumoniae* Y–60 | ≤0.01 | 0.02 | 6.25 | 3.12 |
| *Serratia marcescens* T–26 | 0.02 | 0.4 | 100 | >100 |
| *Serratia marcescens* T–55 | 0.05 | 0.02 | 25 | >100 |
| *Proteus mirabilis* 1287 | 0.05 | 0.1 | 25 | 12.5 |
| *Proteus vulgaris* 6897 | ≤0.01 | 0.1 | 25 | 25 |
| *Proteus morganii* KY 4298 | ≤0.01 | 0.05 | 12.5 | >100 |
| *Proteus rettgeri* KY 4289 | ≤0.01 | <0.01 | 6.25 | 25 |
| *Pseudomonas aeruginosa* #1 | 0.78 | 12.5 | >100 | >100 |
| *Pseudomonas aeruginosa* 145 | 6.25 | 12.5 | >100 | >100 |
| *Pseudomonas putida* 264 | 0.02 | 0.02 | 12.5 | >100 |

* A : The compound obtained in Example 3.

B : The compound obtained in Example 8.

C : The compound obtained in Example 12.

Example 6

Preparation of the sodium salt of (4S, 6R, 7S)-7-[2-(2-amino-4-thiazolyl)-2-syn-methoxyiminoacetamido]-4-hydroxy-1-azabicyclo[4,2,0]oct-2-en-8-on-2-carboxylic acid:

In this Example, 131 mg (0.344 m mole) of the crystals obtained as in Example 3 is suspended in 1.5 ml of water. To the mixture is added 28 mg (0.333 m mole) of sodium hydrogencarbonate and the mixture is stirred at room temperature for 4 hours. The obtained solution is passed through a column packed with 4.5 ml of Diaion HP—20AG and elution is carried out with water. The eluate is collected in 3.3 ml fractions. Fraction Nos. 20 to 28 are concentrated and the concentrate is again passed through a column packed with 25 ml of Sephadex LH 20. Elution is carried out with water and the eluate is collected in 1.4 ml fractions.

Fraction Nos. 12 to 15 are concentrated to obtain crystals which are dried in vacuo at 40°C for 12 hours. Yield 105 mg (76%). Properties of the product are as follows.

Melting point: The compound turns brown at 165°C and the color gradually becomes darker.

$[\alpha]_D^{20°}$: −1° (water, c=1.3)

IR(KBr)$\nu_{max}^{cm^{-1}}$: 3400, 1760, 1665, 1600

NMR ($D_2O$)$\delta$: 6.97 (1H, s), 6.14 (1H, d, J=5.4Hz), 5.61 (1H, d, J=5.1Hz), 4.47—4.56 (1H, m), 3.97—4.21 (1H, m), 3.98 (3H, s), 1.53—2.20 (2H, m)

| | | | |
|---|---|---|---|
| Elementary analysis | | | |
| Found | C: 40.15%, | H: 3.74%, | N: 16.61% |
| Calculated as $C_{14}H_{14}N_5O_6S$ Na | C: 39.91%, | H: 3.83%, | N: 16.62% |

Example 7

Preparation of (4S, 6R, 7S)-7-[2-(2-amino-4-thiazolyl)-2-syn-(2-carboxyprop-2-oxyimino)acetamido]-4-hydroxy-1-azabicyclo[4,2,0]oct-2-en-8-on-2-carboxylic acid:

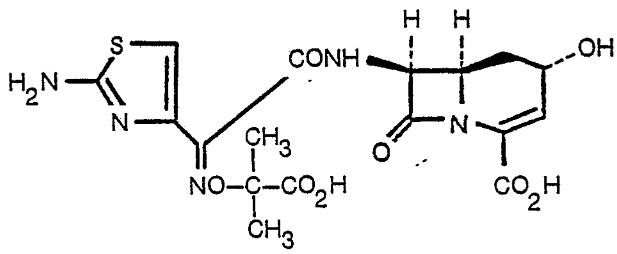

In this Example, 159 mg (0.763 m mole) of phosphorus pentachloride is dissolved in 7.4 ml of anhydrous methylene chloride and the solution is cooled to 0°C.

To the solution is added 404 mg (0.706 m mole) of 2 - (2 - triphenylmethylaminothiazol - 4 - yl) - 2 - syn - (2 - t - butyloxycarbonyl-prop - 2 - oxyimino) acetic acid, followed by stirring at 0°C for 20 minutes to obtain a pale yellow solution. To the solution is added 0.233 ml (1.67 m mole) of triethylamine and the mixture is stirred at 0°C for 5 minutes. After the reaction mixture is concentrated under reduced pressure and dried *in vacuo,* 3.6 ml of anhydrous tetrahydrofuran is added to make an acid chloride solution.

70 mg (0.353 m mole) of (4S, 6R, 7S)-7-amino-4-hydroxy-1-azabicyclo[4,2,0]oct-2-en-8-on-2-carboxylic acid is dissolved in a mixture of 8.4 ml of water and 4.9 ml of tetrahydrofuran and the solution is adjusted to pH 9.8 with triethylamine. The pH of the solution is kept at 8 to 10 with triethylamine and the above acid chloride solution is added dropwise thereto. The mixture is stirred under ice cooling for one hour and 15 minutes and concentrated under reduced pressure to remove the tetrahydrofuran. The concentrate is adjusted to pH 1 to 2 with 2N aqueous hydrochloric acid, saturated with sodium chloride and extracted with 30 ml of ethyl acetate three times. The ethyl acetate extract is washed with saturated saline solution, dried with anhydrous sodium sulfate and concentrated under reduced pressure to obtain 507 mg of a yellow solid. The concentrate is dried *in vacuo* and 5 ml of trichloroacetic acid is added. The mixture is allowed to stand at room temperature for 10 minutes. To the mixture is added 3 ml of water and the mixture is again allowed to stand for 10 minutes.

The mixture is concentrated under reduced pressure and the resulting yellow slurry is purified by column chromatography using 65 ml of Diaion HP—10 treated with dimethylsulfoxide.

Elution is carried out with 65 ml of water, 300 ml of a mixture of water and methanol (10:1) and 420 ml of a mixture of water and methanol (6:1) to obtain fractions 1 to 25, 26 to 60 and 61 to 130, respectively. Fraction Nos. 42 to 95 are combined and concentrated under reduced pressure to obtain 35.3 mg of a crude product. The product is purified by column chromatography using 7 ml of Diaion HP—10.

Elution is carried out with 20 ml of water, 40 ml of a mixture of water and methanol (20:1), 40 ml of a mixture of water and methanol (15:1), 100 ml of a mixture of water and methanol (10:1) to obtain fractions 1 to 15, 16 to 43, 44 to 70 and 71 to 136, respectively. Fraction Nos. 26 to 90 are combined and concentrated under reduced pressure to obtain 22.1 mg (13.8%) of the desired product.

Properties of the product are as follows.

IR$\nu_{max}^{cm^{-1}}$ (KBr): 1770, 1660, 1635

NMR (DMSO-$d_6$) $\delta$: 9.28 (1H, d, J=8.3Hz), 7.26 (3H, s), 6.73 (1H, s), 6.33 (1H, d, J=4.9Hz), 5.53 (1H, dd, J=5.0, 8.3Hz), 5.3—5.4 (1H, m), 4.3—4.4 (1H, br), 3.8—4.0 (1H, m), 1.6—2.0 (2H, m), 1.41 (6H, s)

$[\alpha]_D^{24°} = -24.4°$ (methanol, c=1.0)

Example 8

Preparation of (±)-cis-7β-[2-(2-amino-4-thiazolyl)-2-syn-methoxyiminoacetamido]-4α-hydroxy-1-azabicyclo[4,2,0]oct-2-en-8-on-2-carboxylic acid

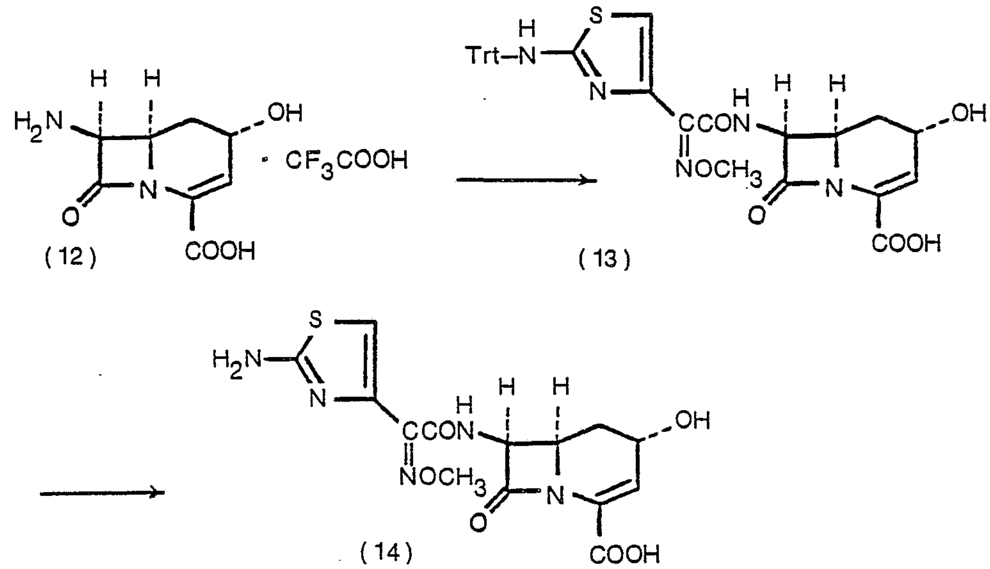

(12) (13) (14)

In this Example, 60 mg (0.203 m mole) of the trifluoroacetate of (±)-cis-7β-amino-4α-hydroxy-1-azabicyclo[4,2,0]oct-2-en-8-on-2-carboxylic acid obtained as in the method described in Reference Example 5 is dissolved in a mixture of 1 ml of water and 1 ml of tetrahydrofuran and 56 μl of triethylamine is added to the solution.

The mixture is referred to as Reaction solution A. On the other hand, 125.7 mg (0.280 m mole) of 2-(2-tritylamino-4-thiazolyl)-2-syn-methoxyiminoacetic acid is dissolved in 1.2 ml of anhydrous methylenechloride and the solution is allowed to react under cooling on a dry-ice-carbon tetrachloride bath with stirring for 50 minutes after the addition of 39.3 μl of triethylamine and 55 mg (0.264 m mole) of phosphorous pentachloride. The reaction solution is concentrated under reduced pressure and to the residue is added 1 ml of anhydrous tetrahydrofuran to make an acid chloride solution. The acid chloride solution with 28 μl of triethylamine are added to Reaction solution A obtained above in three portions in 5 minutes. The mixture is allowed to react additionally for 35 minutes and adjusted to pH 2.5 with 10% citric acid. The solution is saturated with sodium chloride and extracted with ethyl acetate three times. The layers of the organic solvent are washed with saturated saline solution and dried with anhydrous sodium sulfate. After filtration and concentration, the residue is subjected to purification by silica gel chromatography (silica gel 15 g, solvent, methanol:chloroform = 1:3) to obtain 185.5 mg of a crude acyl product. To 86 mg of the acyl product [Compound (13)] 3 ml of 50% acetic acid is added and the mixture is stirred at a temperature of 50°C for 50 minutes. The mixture is cooled and the deposited triphenylcarbinol is removed by filtration. The filtrate is concentrated under reduced pressure to obtain a yellow glassy crude product. The product is subjected to purification using 8 ml of Diaion HP—20 (product of Mitsubishi Kasei Kogyo Co., Ltd.) and a solvent of methanol and water (1:9 to 2:1 by volume, the same shall apply hereinafter) to obtain 14.5 mg (44.3%) of the desired compound. Properties of the compound are as follows.

$IR\nu_{max}^{KBr}$ ($cm^{-1}$): 3450, 1775, 1670, 1635, 1550

NMR (DMSO $d_6$—$CD_3OD$)δ: 9.28 (1H, d, J=8.4Hz), 7.07 (2H, s), 6.75 (1H, s), 6.27 (1H, d, J=5.4Hz), 5.56 (2H, m), 2.77—2.04 (2H, m)

The signal of —$OCH_3$ is superimposed with the signals due to the solvent.

Example 9

Preparation of (±)-cis-7β-phenylacetamido-4α-hydroxy-1-azabicyclo[4,2,0]oct-2-en-8-on-2-carboxylic acid:

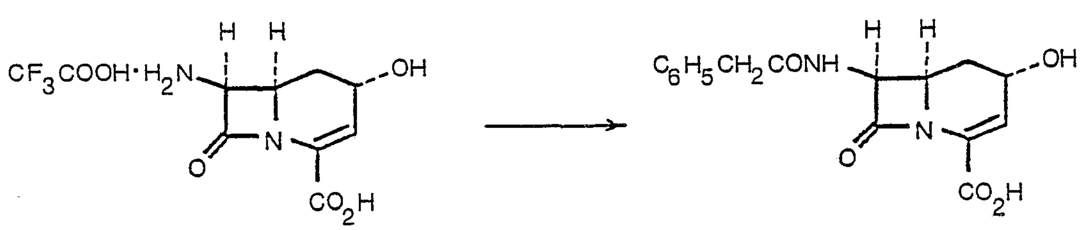

In this Example, 194.6 mg (0.623 m mole) of the trifluoroacetate of (±)-cis-7β-amino-4α-hydroxy-1-azabicyclo[4,2,0]oct-2-en-8-on-2-carboxylic acid is dissolved in a mixture of 3.1 ml of water and 6.2 ml of acetone and 209 mg (2.49 m mole) of sodium hydrogencarbonate are added to the solution. Then, 125 mg (0.810 m mole) of phenylacetylchloride in 2 ml of acetone is added dropwise to the mixture with stirring under ice cooling. 10.5 mg (0.068 m mole) and 17.6 mg (0.144 m mole) of phenylacetylchloride are added to the mixture after 1.5 and 2.5 hours, respectively. After 2 hours and 45 minutes, the reaction solution is concentrated under reduced pressure to remove acetone. Water (10 ml) and 1 N hydrochloric acid (1 ml) are added to the concentrate and the resulting solution is extracted three times with 20 ml of ethyl acetate. The ethyl acetate layer is washed with saturated saline solution, dried with anhydorus sodium sulfate, subjected to filtration and concentrated under reduced pressure.

The obtained brown oily product is triturated with ether, subjected to filtration and dried to obtain 120 mg (60.4%) of a powder of the desired compound. Properties of the product are as follows.

IR$\nu_{max}^{KBr}$ (cm$^{-1}$): 3400, 1780, 1670, 1640
NMR ($CD_3OD$)δ: 7.27 (5H, s), 6.39 (1H, d, J=5.4Hz), 5.46 (1H, d, J=5.1Hz), 4.37 (1H, m), 4.01 (1H, m), 3.57 (2H, s), 2.0—1.1 (2H, m)

Example 10

Preparation of (±)-cis-7β-[2-(2-amino-4-thiazolyl)-2-syn-methoxyiminoacetamido]-4β-hydroxy-1-azabicyclo[4,2,0]oct-2-en-8-on-2-carboxylic acid:

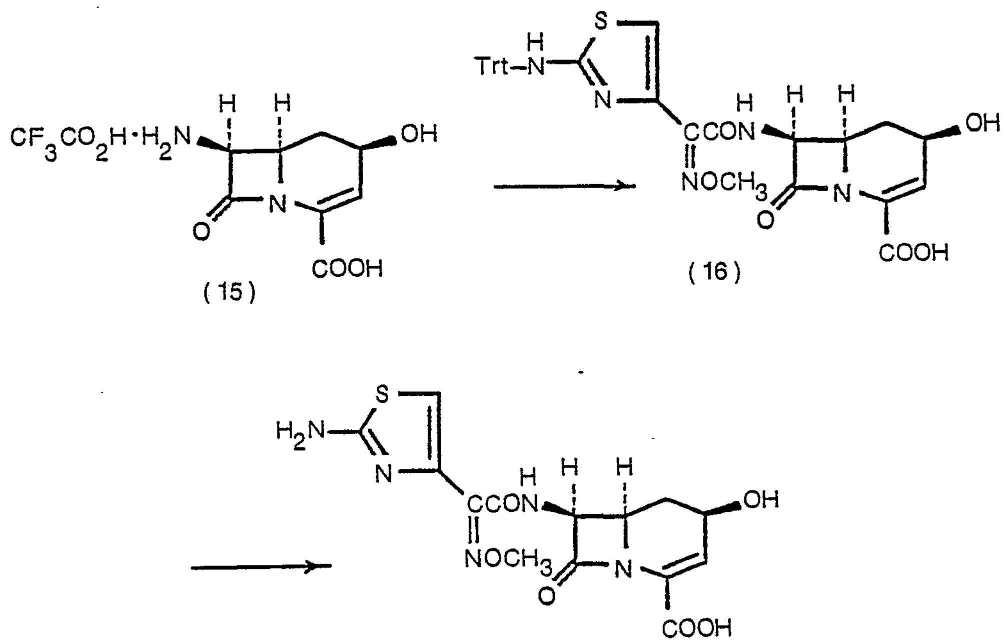

(15) (16)

In this Example, 127 mg (0.286 m mole) of 2-(2-tritylamino-4-thiazolyl)-2-syn-methoxyimino-acetic acid is dissolved in 1.3 ml of anhydrous dichloromethane and 29 mg (0.286 m mole) of triethylamine is added at a temperature of −20°C. Then after adding 60 mg (0.286 m mole) of phosphorus pentachloride, the mixture is stirred at a temperature of −18 to −20°C for 30 minutes. The reaction mixture is concentrated under reduced pressure and lyophilized for 30 minutes. The residue is dissolved in 3 ml of anhydrous tetrahydrofuran to make an acid chloride solution.

Separately, 63.8 mg (0.204 m mole) of the trifluoroacetate of (±)-cis-7β-amino-4β-hydroxy-1-azabicyclo[4,2,0]oct-2-en-8-on-2-carboxylic acid obained as in Reference Example 9 is suspended in a mixture of 1.3 ml of tetrahydrofuran and 1.3 ml of water and 103 mg (1.02 m mole) of triethylamine is added. To the mixture is added the acid chloride solution dropwise with stirring under ice cooling and 22 mg (0.22 m mole) of triethylamine is added. The mixture is allowed to react for 4 hours and 45 minutes with stirring under ice cooling. Then, after adding 2 ml of 1N—HCl, the reaction mixture is extracted three times with 20 ml of ethyl acetate. The extract is dried with anhydrous sodium sulfate, and concentrated under reduced pressure to obtain the trityl-protected crude product [Compound (16)] of the desired compound. The product is dissolved in 20 ml of 50% acetic acid and the solution is stirred at a temperature of 50 to 60°C on a bath for one hour. After cooling to room temperature, the reaction solution is subjected to filtration under reduced pressure. The filtrate is concentrated under reduced pressure to obtain 173 mg of a crude product of the desired compound.

The product is subjected to purification using 40 ml of Diaion HP—20AG (product of Mitsubishi Kasei Kogyo Co., Ltd.) and a solvent of water and methanol (10:1 to 1:1). 32 mg (42%) of a pale yellow

powder of the desired compound is obtained. Properties of the product are as follows.

$IR\nu_{max}^{KBr}$ $(cm^{-1})$: 3475, 1780, 1680, 1670, 1655, 1645

NMR $(DMSO-d_6)\delta$: 9.33 (1H, d, J=8.8Hz), 7.18 (2H, s), 6.75 (1H, s), 6.06 (1H, d, J=1.4Hz), 5.38 (2H, one H is q, J=8.8, 5.4Hz), 4.50 (1H, m), 3.98 (1H, m), 3.84 (3H, s), 2.17—1.37 (2H, m)

Example 11

Preparation of (±)-cis-7β-[(R)-2-t-butyloxycarbonyl-amino-2-phenylacetamido]-4α-hydroxy-1-azabicyclo[4,2,0]oct-2-en-8-on-2-carboxylic acid, t-butyl ester:

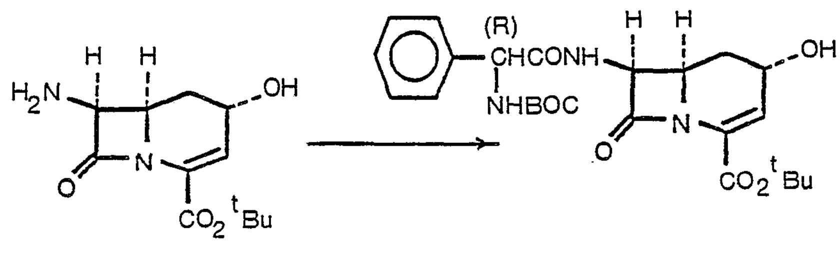

In this Example, 23.8 mg (0.095 m mole) of (R)-N-t-butyloxycarbonylphenylglycine is dissolved in 1 ml of anhydrous tetrahydrofuran, and 0.095 ml (0.095 m mole) of 1N-N-methylmorpholine-tetrahydrofuran and 0.095 ml (0.095 m mole) of 1 N-isobutyl chloroformate-tetrahydrofuran are added at a temperature of −30°C. The mixture is stirred for 30 minutes and 20 mg (0.079 m mole) of (±)-cis-7β-amino-4α-hydroxy-1-azabicyclo[4,2,0]oct-2-en-8-on-2-carboxylic acid, t-butyl ester obtained as in Reference Example 4 in 1 ml of anhydrous methylene chloride is added thereto. The mixture is allowed to react at a temperature of −30°C for 45 minutes and at 0°C for 4 hours. The reaction mixture is then diluted with 5 ml of methylene chloride and is washed successively with water, 1N—HCl, 5%—$NaHCO_3$, water and saturated sodium chloride solution. The washing is dried with anhydrous sodium sulfate and concentrated to obtain 51 mg of a crude acyl-compound. Purification by silica gel chromatography with 5 g of silica gel and a solvent of n-hexane and ethyl acetate (1.5:1) is carried out to obtain 10 mg of the more polar isomer, 8 mg of the less polar isomer and 4 mg of a mixture thereof. Total yield 22 mg (57%). Properties of the compounds are as follows.

The more polar isomer $IR\nu_{max}^{CHCl_3}$ $(cm^{-1})$: 3430, 1780, 1725(sh), 1717, 1697, 1630

The less polar isomer $IR\nu_{max}^{CHCl_3}$ $(cm^{-1})$: 3430, 1780, 1722(sh), 1715, 1695, 1630

Example 12

Preparation of cis-7β-[(R)-2-phenylglycinamido-4α-hydroxy-1-azabicyclo[4,2,0]oct-2-en-8-on-2-carboxylic acid:

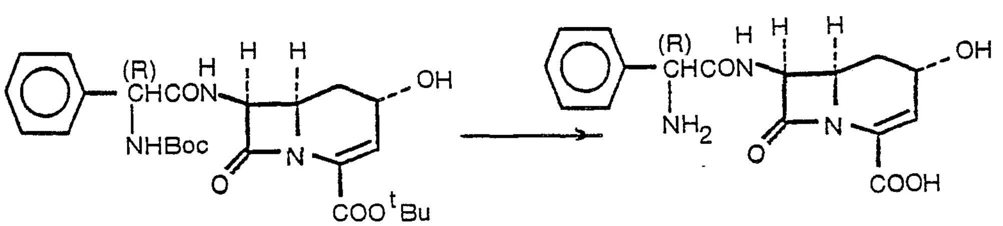

In this Example, 56 mg (0.118 m mole) of the less polar isomer of cis-7β-[(R)-2-t-butyloxycarbonylamino-2-phenylacetamido]-4α-hydroxy-1-azabicyclo[4,2,0]oct-2-en-8-on-2-carboxylic acid, t-butyl ester obtained as in Example 11 is dissolved in a mixture of 1 ml of anhydrous methylene chloride and 1 ml of anisole, and 2 ml of trifluoroacetic acid is added under ice cooling. The mixture is allowed to stand for 4 hours under ice cooling and then concentrated under reduced pressure. After adding dry benzene, the concentrate is again concentrated to obtain an oily product. The product is triturated with ether and the resulting precipitate is recovered by filtration to obtain 42.1 mg (80%) of a pale yellow powder of the trifluoroacetate of the desired compound. Properties of the product are as follows.

$IR\nu_{max}^{KBr}$ $(cm^{-1})$: 3485, 1880(sh), 1870, 1700(sh), 1685, 1635

The obtained trifluoroacetate is dissolved in 2 ml of 1M-phosphate buffer (pH 7.0) and subjected to purification using 50 ml of Diaion HP—20AG and a solvent of water to water and methanol (9:1). The purified solution is lyophilized to obtain 28 mg (72%) of the desired compound. Properties of the product are as follows.

$[\alpha]_D^{24^\circ} = -26.0$ (c=0.53, $H_2O$)

$IR\nu_{max}^{KBr}$ ($cm^{-1}$): 3480, 1780, 1770, 1680—1705, 1570—1650

NMR ($D_2O$)$\delta$: 7.52 (5H, s), 6.03 (1H, d, J=5.4Hz), 5.5 (1H, d, J=5.1Hz), 5.21 (1H, s), 4.28 (1H, m), 4.06—3.85 (1H, m), 1.76—0.99 (2H, m)

Example 13

Preparation of cis-7$\beta$-[(R)-2-phenyl-2-(4-ethyl-2,3-dioxo-1-piperadinylcarbonylamino)acetamido]-4$\alpha$-hydroxy-1-azabicyclo[4,2,0]oct-2-en-8-on-2-carboxylic acid:

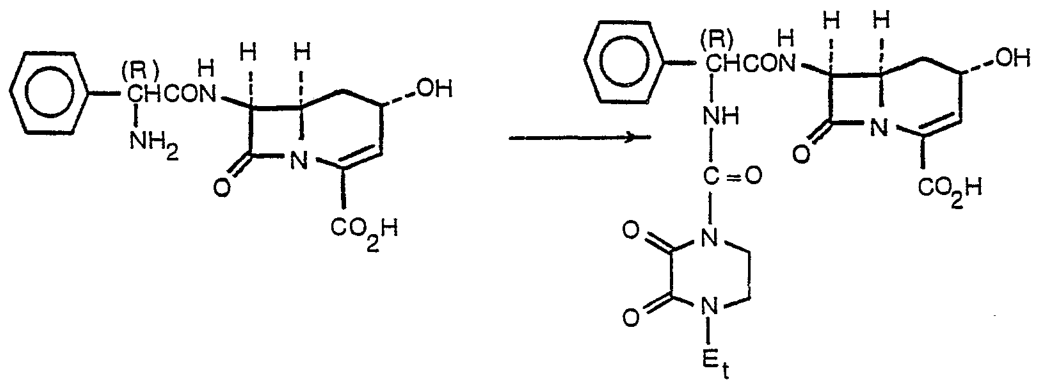

In this Example, 10 mg of cis-7$\beta$-[(R)-2-phenylglycinamido]-4$\alpha$-hydroxy-1-azabicyclo[4,2,0]oct-2-en-8-on-2-carboxylic acid obtained as in Example 12 is disolved in a mixture of 180 $\mu$l of acetone and 50 $\mu$l of water and a solution of 9.2 $\mu$l (0.066 m mole) of triethylamine and 8.0 mg (0.039 m mole) of 4-ethyl-2,3-dioxo-1-piperadinyl-carboxylic acid chloride in 100 $\mu$l of anhydrous tetrahydrofuran is added under ice-cooling to the solution.

The mixture is stirred for 3 hours and 50 $\mu$l of 20% aqueous phosphoric acid is added to the reaction mixture.

The organic solvent is removed by distillation to obtain an aqueous solution containing the desired product. The solution is subjected to the purification using 7 ml of HP—20AG and a solvent of water and methanol (10:1 to 1:2) to obtain 9.8 mg (65%) of a pale yellow desired product.

Properties of the product are as follows.

$[\alpha]_D^{28°} = -57.0°$ (c=0.1, methanol)

$IR\nu_{max}^{KBr}$ ($cm^{-1}$): 3475, 1770, 1760, 1715, 1670—1690

NMR ($D_2O$)$\delta$: 7.47 (5H, s), 6.21 (1H, d, J=5.4Hz), 5.47 (1H, s), 5.45 (1H, d, J=5.1Hz), 4.30 (1H, m), 3.32—4.08 (7H, m), 0.99—1.68 (2H, m), 1.18 (3H, t, J=7.2Hz)

Antimicrobial activities of the compound obtained in Examples 8, 10, 12 and 13 are as follows. Heart infusion Agar Dilution Method (pH 7.2) is employed. Cefazolin is used as a control.

Example 14

Preparation of (±)-cis-7$\beta$-[2-(2-amino-5-bromo-4-thiazolyl)-2-syn-methoxyiminoacetamido]-4$\alpha$-hydroxy-1-azabicyclo[4,2,0]oct-2-en-8-on-2-carboxylic acid:

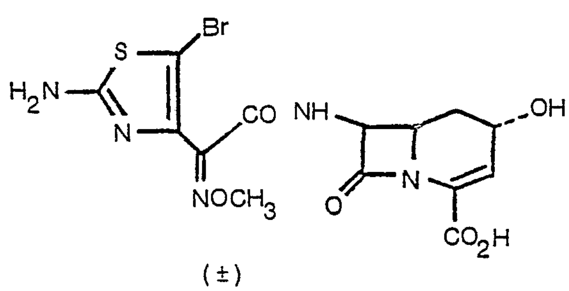

(±)

In this Example, 72.3 mg (0.348 m mole) of phosphorus pentachloride is added to the solution of 182 mg (0.348 m mole) of 2-(2-tritylamino-5-bromo-4-thiazolyl)-2-syn-methoxyiminoacetic acid and 48 $\mu$l (0.348 m mole) of triethylamine in 2 ml of tetrahydrofuran with stirring at −20°C and the mixture is stirred for 40 minutes. The solution is added dropwise to a mixture of 46 mg (0.232 m mole) of (±)-cis-7$\beta$-amino-4$\alpha$-hydroxy-1-azabicyclo[4,2,0]oct-2-en-8-on-2-carboxylic acid and 79 $\mu$l (0.58 m mole) of triethylamine in 6 ml of 50% aqueous tetrahydrofuran with stirring under ice cooling. The mixture is allowed to react for two hours under ice cooling, adjusted to pH 2.0 with 1N hydrochloric acid and extracted twice with 10 ml of ethyl acetate.

The organic layers are combined, washed with 20 ml of saturated saline solution and dried over sodium sulfate. After evaporation under reduced pressure, the residue is dissolved in a mixture of 9 ml of methanol and 1 ml of water. The solution is adjusted to pH 1.0 with 1N hydrochloric acid and stirred for one hour at room temperature to remove the trityl group. After adjusting the pH to 7 with triethylamine, 10 ml of water is added to the reaction mixture. Then, the solution is washed with 10 ml of ethyl acetate. The aqueous layer is adjusted to pH 2.0 with 1N hydrochloric acid and charged on a column packed with 30 ml of Diaion HP—10. Elution is carried out with a mixture of water and methanol (4:1 to 2:1). Fractions showing an Rf value of 0.45 by silica gel thin layer chromatography (solvent:butanol:acetic acid:water = 4:1:1) are combined and evaporated under reduced pressure to give 54 mg of a white powder (yield 50.5%). Properties of the product are as follows:

IR $\nu$max(KBr) $cm^{-1}$: 1780, 1770, 1670, 1630, 1540

NMR ($CD_3OD$) $\delta$: 6.44 (1H, d, J=5.4Hz), 5.59 (1H, d, J=5.1Hz), 4.4 (1H, m), 4.1 (1H, m), 3.98 (3H, s), 2.8—1.8 (2H, m)

## Example 15

Preparation of (4S, 6R, 7S)-7-[2-(2-amino-5-bromo-4-thiazolyl)-2-syn-methoxyiminoacetamido]-4-hydroxy-1-azabicyclo[4,2,0]oct-2-en-8-on-2-carboxylic acid:

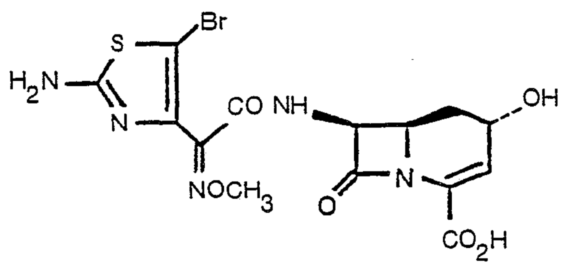

In this Example, 170 mg (0.356 m mole) of 2-(2-tritylamino-5-bromo-4-triazolyl)-2-syn-methoxyiminoacetic acid is dissolved in 2 ml of anhydrous tetrahydrofuran and 49 $\mu$l (0.356 m mole) of triethylamine is added at −20°C. After adding 74 mg (0.356 m mole) of phosphorus pentachloride, the mixture is allowed to react with stirring at −20°C for one hour.

Separately, 32 mg (0.162 m mole) of (4S, 6R, 7S)-7-amino-4-hydroxy-1-azabicyclo[4,2,0]oct-2-en-8-on-2-carboxylic acid is dissolved in a mixture of 2 ml of tetrahydrofuran and 2 ml of water and 71 $\mu$l (0.518 m mole) of triethylamine is added. The acid chloride solution prepared above is added dropwise to the solution with stirring under ice cooling. The mixture is allowed to react for two hours under ice cooling. Then, the mixture is adjusted to pH 2.0 with 1N hydrochloric acid and extracted twice with 10 ml of ethyl acetate. The organic layers are washed with 20 ml of saturated saline solution, dried over sodium sulfate and concentrated under reduced pressure. The residue is dissolved in 10 ml of 50% aqueous acetic acid and stirred at 50°C for 30 minutes. After concentrating, the residue is dissolved in 10 ml of water and the solution is washed with 10 ml of ethyl acetate. After adjusting the pH to 2 with 1N hydrochloric acid, the aqueous layer is charged on a column packed with 20 ml of Diaion HP—10 and elution is carried out with a mixture of water and methanol (4:1 to 2:1).

Fractions showing an Rf value of 0.45 by silica gel thin layer chromatography (solvent:butanol:-acetic acid:water = 4:1:1) are combined and concentrated under reduced pressure to give 34.2 mg of a white powder (yield 46%).

Properties of the product are as follows:

IR $\nu$max(KBr) $cm^{-1}$: 1780, 1770, 1670, 1630, 1540

NMR ($CD_3OD$)$\delta$: 6.44 (1H, d, J=5.4Hz), 5.59 (1H, d, J=5.1Hz), 4.4 (1H, m), 4.1 (1H, m), 3.98 (3H, s), 2.8—1.8 (2H, m)

$[\alpha]_D^{24°} = -35.6°$ (c=0.5, methanol)

| Microorganism | Minimum Innibitory Concentration ($\mu$g/ml) 8* | 10* | 12* | 13* | Cefazolin |
|---|---|---|---|---|---|
| *Staphylococcus aureus* 209–P | 12.5 | 50 | 3.12 | 1.56 | 0.02 |
| *Staphylococcus aureus* Smith | 25 | 50 | 25 | 6.25 | 0.78 |
| *Staphylococcus epidermidis* | 50 | >100 | 12.5 | 6.25 | 0.78 |
| *Escherichia coli* NIHJC–2 | 0.05 | 0.2 | 6.25 | 1.56 | 1.56 |
| *Escherichia coli* GN2411–5 | 0.02 | 0.2 | 3.12 | 0.78 | 0.78 |
| *Escherichia coli* Juhl | 0.05 | 0.1 | 3.12 | 3.12 | 1.56 |
| *Klebsiella pneumoniae* 8045 | 0.02 | 0.05 | 3.12 | 0.2 | 1.56 |
| *Klebsiella pneumoniae* Y–60 | 0.02 | 0.2 | 6.25 | 25 | 3.12 |
| *Serratia marcescens* T–26 | 0.4 | 3.12 | 100 | 12.5 | >100 |
| *Serratia marcescens* T–55 | 0.02 | 0.78 | 25 | 6.25 | >100 |
| *Proteus mirabilis* 1287 | 0.1 | 0.2 | 25 | 6.25 | 12.5 |
| *Proteus vulgaris* 6897 | 0.1 | 0.05 | 25 | 0.02 | 25 |
| *Proteus morganii* KY 4298 | 0.05 | 0.1 | 12.5 | 0.4 | >100 |
| *Proteus rettgeri* KY 4289 | <0.01 | 0.02 | 6.25 | 6.25 | 25 |
| *Pseudomonas aeruginosa* #1 | 12.5 | >100 | >100 | 50 | >100 |
| *Pseudomonas aeruginosa* 145 | 12.5 | >100 | >100 | 100 | >100 |
| *Pseudomonas putida* 264 | 0.02 | 0.1 | 12.5 | 0.2 | >100 |

* 8, 10, 12 and 13 represent the compounds obtained in Examples 8, 10, 12 and 13, respectively.

Reference Example 1

Preparation of (±)-cis-7-azido-1-azabicyclo[4,2,0]oct-2-en-8-on-2-carboxylic acid, t-butyl ester:

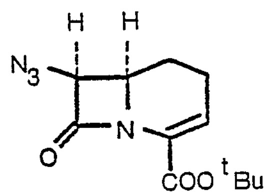

The present compound is produced through the following Processes 1 and 2.

1) Preparation of 2-[4-(3-butenyl)-3-azido-2-oxo-azetidin-1-yl]-2-diethylphosphonoacetate, t-butyl ester:

In this Example, 447 mg (1.78 m mole) of t-butyl $\alpha$-aminodiethylphosphonoacetate is dissolved in 25 ml of anhydrous ether and 164 mg (1.96 m mole) of 4-pentene-1-al is added to the solution. The solution is stirred at room temperature for one hour and 200 mg of Molecular Sieve (4A) (the product of

Wako Junyaku Co., Ltd., the same molecular sieve is used hereinafter) and 150 mg of anhydrous magnesium sulfate are added to the solution. The mixture is stirred for one hour.

The reaction mixture is subjected to filtration under reduced pressure and the filtrate is concentrated under reduced pressure to obtain a pale yellow oily product. Anhydrous benzene is added to the product and the mixture is concentrated under reduced pressure to obtain a pale yellow oily product. The presence of a shiff's base in the product is confirmed by nuclear magnetic resonance spectrum. The product is dissolved in 12.5 ml of cyclohexane and 12.5 ml of anhydrous benzene, and 0.369 ml (2.66 m mole) of triethylamine and 200 mg of Molecular Sieve are added to the solution. Azidoacetylchloride [319 mg (2.66 m mole)] dissolved in 12.5 ml of cyclohexane is added dropwise to the mixture with stirring at room temperature in 1.5 hours. The reaction mixture is further stirred for 30 minutes and diluted with 10 ml of benzene. The reaction solution is washed with 5% diluted hydrochloric acid, saturated sodium bicarbonate, deionized water and saturated sodium chloride solution, dried with anhydorus sodium sulfate and concentrated under reduced pressure to obtain a brown oily product which is identified as a crude product of the desired compound. The oily product is charged on a column packed with 45 g of silica gel. Elution is carried out with a mixture of n-hexane and ethyl acetate (1:2) to obtain two types of isomers. Properties of the isomers are given below and they are identified as the isomers at the 3- and 4-positions, i.e. 345 mg of cis-isomer and 58 mg of trans-isomer. Total yield is 54.2%.

Cis-isomer

IR$\nu_{max}^{CHCl_3}$ ($cm^{-1}$): 2120, 1775, 1770(sh), 1750, 1740(sh), 1645

NMR ($CDCl_3$)$\delta$: 6.13—6.33 (1H, m), 4.93—5.17 (2H, m), 4.50—4.93 (2H, m), 3.80—4.40 (5H, m), 1.93—2.17 (4H, m), 1.50 (9H, s), 1.33 (6H, t)

Trans-isomer

IR$\nu_{max}^{CHCl_3}$ ($cm^{-1}$): 2120, 1780, 1755, 1750(sh), 1650

NMR ($CDCl_3$)$\delta$: 5.43—6.20 (1H, m), 4.80—5.30 (2H, m), 3.75—4.75 (7H, m), 2.0—2.50 (4H, m), 1.50 (9H, d), 1.17 (6H, m)

2) Preparation of (±)-cis-7-azido-1-azabicyclo[4,2,0]oct-2-en-8-on-2-carboxylic acid, t-butyl ester:

In this Example, 298 mg (0.716 m mole) of 2-[4-(3-butenyl)-3-azido-2-oxoazetidin-1-yl]-2-diethylphosphonoacetate, t-butyl ester obtained in the above 1) is dissolved in 8.5 ml of dioxane and 2.5 ml of deionized water, and 30 mg of osmium tetroxide is added thereto. The solution is stirred for 30 minutes. Powdered sodium periodate [496 mg (2.32 m mole)] is added to the black reaction mixture in 20 minutes.

After stirring for 1.5 hours, the reaction solution is extracted with 50 ml of ether three times. Ether extracts are combined and washed with saturated sodium chloride solution. The resulting solution is dried with anhydrous sodium sulfate and concentrated under reduced pressure to obtain a dark-brown oily product. The product is charged on a column packed with 5 g of silica gel and elution is carried out with a solvent of benzene and ethyl acetate (1:2). Fractions positive to 2,4-dinitrophenylhydrazine reaction are collected and concentrated to obtain 235 mg of an oily product. The oily product is dissolved in 15 ml of anhydrous acetonitrile. Sodium hydride [50%, 27.1 mg (0.563 m mole)] is added to the solution in a stream of nitrogen with stirring at room temperature.

After stirring for 20 minutes, the reaction mixture is poured in 20 ml of 2% aqueous acetic acid and the solution is extracted with 50 ml of ether four times. Ether extracts are combined and washed with saturated sodium chloride solution. The resulting solution is dried with anhydrous sodium sulfate and concentrated under reduced pressure to obtain 180 mg of an oily product. The oily product is charged on a column packed with 5 g of silica gel and elution is carried out with a solvent of n-hexane and ethyl acetate (3.5:1, by volume). White crystals (91 mg) of the desired compound are obtained. Yield 51%.

Properties of the compound are as follows.

Melting point: 64.5—65.5°C

IR$\nu_{max}^{CHCl_3}$ ($cm^{-1}$): 2130, 1790, 1730, 1640

NMR ($CDCl_3$)$\delta$: 6.30 (1H, t, J=4Hz), 4.93 (1H, d, J=5Hz), 3.80 (1H, q), 1.6—2.6 (4H, m), 1.52 (9H, s)

Reference Example 2

Preparation of (±)-cis-7-azido-1-azabicyclo[4,2,0]oct-2-en-8-on-2-carboxylic acid, t-butyl ester:

This Example shows an alternative method for preparing the compound in Reference Example 1. The present compound is prepared according to the following processes a) and b).

a) Preparation of (±)-cis-7-azido-1-azabicyclo[4,2,0]oct-2,4-diene-8-on-2-carboxylic acid, t-butyl ester:

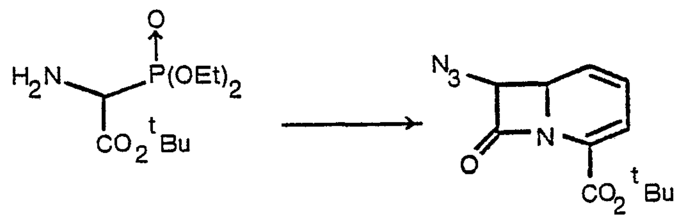

1) Preparation of azidoacetyl chloride solution:

Azidoacetic acid [5 g (49.5 m moles)] is dissolved in 37.5 ml of methylene chloride and, with stirring at room temperature, 5.96 g (5.01 m moles) of thionyl chloride in 12.5 ml of methylene chloride is added dropwise thereto. Then, the mixture is refluxed for 6.5 hours under heating. After allowing to cool, 150 ml of methylene chloride is added thereto to use for the following reaction.

2) t-Butyl $\alpha$-aminodiethylphosphonoacetate [7.9 g (29.6 m moles)] and 4.6 g (35.3 m moles) of 4,4-dimethoxy-2-butenal are dissolved in 250 ml of methylene chloride, and 5.0 g of anhydrous magnesium sulfate powder and 3.85 g of Molecular Sieves 4A are added thereto under stirring.

The resulting mixture is reacted for 3.5 hours at room temeprature. Then, magnesium sulfate and the molecular sieves are removed by filtration, and the solution is concentrated under reduced pressure to obtain an oily Schiff's base. This is dissolved in 312.5 ml of methylene chloride and, after adding thereto 5.08 g (50.2 m moles) of triethylamine, the azidoacetyl chloride solution prepared in step 1) is added dropwise thereto in one hour under ice cooling.

After further continuing the reaction for one hour, the reaction solution is washed successively with saturated sodium bicarbonate aqueous solution and saturated sodium chloride aqueous solution, dried with anhydrous sodium sulfate, and concentrated under reduced pressure. The thus obtained crude acetal compound is dissolved in 150 ml of acetone, 2.5 g (13.1 m moles) of p-toluenesulfonic acid monohydrate is added thereto, and the reaction is conducted at room temperature for 2 hours. Then, 300 ml of ethyl acetate is added thereto, and the solution is washed with 150 ml of saturated sodium bicarbonate aqueous solution.

The washing is extracted with ethyl acetate, and the ethyl acetate extract is combined, washed with saturated sodium chloride aqueous solution, and then dried with anhydrous sodium sulfate. After removal of anhydrous sodium sulfate by filtration, the filtrate is concentrated under reduced pressure to obtain a crude aldehyde compound. This is dissolved in 125 ml of benzene, 3.5 g (31.3 m moles) of diazabicyclo [2,2,2] octane is added thereto, and the reaction is conducted overnight at room temperature.

To the reaction solution, 150 ml of ethyl acetate is added, and the resulting solution is washed with 100 ml of saturated ammonium chloride aqueous solution, dried with anhydrous sodium sulfate, and concentrated under reduced pressure.

The thus obtained black oil is charged on a column packed with 120 g of silica gel, and elution is carried out using a mixture of ethyl acetate and n-hexane (1:3) as an eluent to purify. Concentration of fractions 9—23 (one fraction: 18 ml) under reduced pressure yields 3.2 g (yield: 40.5% based on t-butyl $\alpha$-aminodiethylphosphonoacetate) of the end product as crystals. Properties of this compound are as follows.

Melting point: 68.0—69.2°C

IR$\nu_{max}^{CHCl_3}$ ($cm^{-1}$): 2130, 1790, 1720, 1630

NMR ($CDCl_3$)$\delta$: 6.64 (d, 1H, J=6Hz), 6.24 (ddd, 1H, J=2.5, 6.0, 6.0Hz), 6.04 (dd, 1H, J=2.0, 10.0Hz), 5.26 (d, 1H, J=5.0Hz), 4.64 (m, 1H), 1.50 (s, 9H)

a') Preparation of (±)-cis-7-azido-1-azabicyclo[4,2,0]oct-2,4-diene-8-on-2-carboxylic acid, t-butyl ester:

An improvement of the above method 2)

In this Example, 2.67 g of t-butyl $\alpha$-amidinodiethylphosphono acetate and 2.00 g of 4,4-dimethoxy-2-butenal are dissolved in 15 ml of anhydrous methylene chloride. The solution is charged on a column packed with 35 ml of Molecular Sieve (4A, 1/16) and elution is carried out with 80 ml of methylene chloride to obtain a solution containing a Shiff's base. 5.57 ml of triethylamine, 2.02 g of azidoacetic acid and 1.85 g of Molecular Sieve powder are added to the solution. After adding 2.30 g of phosphorus oxychloride in 15 ml of methylene chloride under ice-cooling in one hour, the mixture is allowed to react for one hour. 20 ml of acetone and 3.0 g of p-toluenesulfonic acid monohydrate are

added and the mixture is allowed to stand overnight. The mixture is washed with saturated sodium bicarbonate and saturated saline solution, dried with anhydrous sodium sulfate and concentrated to obtain an oily product. The product is dissolved in 80 ml of anhydrous benzene and 3.0 g of DABCO®

( 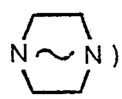 );

catalyst, based on triethylenediamine is added. The mixture is allowed to react for four hours. The reaction mixture is washed with saturated ammonium chloride and saturated saline solution, dried and concentrated. The concentrate is subjected to silica gel chromatography (100 ml of silica gel, solvent, n-hexane:ethyl acetate = 3:1). The eluate of fraction Nos. 6 to 18 (one fraction: 15 ml) are concentrated to obtain 1.57 g (60.4%) of an orange desired compound.

Properties of the compound agree well with those of the compound obtained in Reference Example 2-a).

b) Preparation of (±)-cis-7-azido-1-azabicyclo[4,2,0]oct-2-en-8-on-2-carboxylic acid, t-butyl ester:

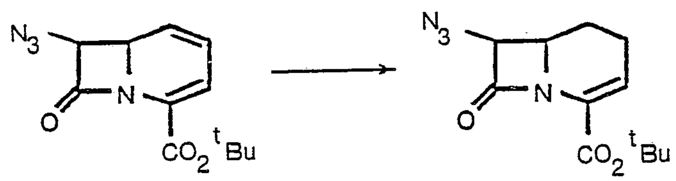

In this example, 5.45 g of the diene compound obtained as in Reference Example 2-a) or 2-a') is dissolved in 360 ml of ethyl acetate and 900 mg of 10% palladium-carbon is added. The mixture is subjected to catalytic reduction at room temperature in a stream of hydrogen for 55 minutes. Then, the catalyst is removed by filtration and the filtrate is washed with 100 ml of 10% aqueous citric acid and 100 ml of saturated saline solution. The washing is dried with anhydrous sodium sulfate and concentrated under reduced pressure. The concentrate is subjected to liquid chromatography at 25 kg/cm$^2$ using 500 cm$^3$ of Prepak silica gel column (product of Waters Co.) to separate the unreacted starting compound and to obtain 2.58 g (47.0%) of the desired compound. The following properties of the desired compound agree well with those of the compound obtained in Reference Example 1.

Melting point: 64.5—65.5°C

IR$\nu_{max}^{CHCl_3}$ (cm$^{-1}$): 2130, 1790, 1730, 1640

NMR ($CDCl_3$)$\delta$: 6.30 (1H, t, J=4.0Hz), 4.93 (1H, d, J=5.0Hz), 3.80 (1H, q), 1.6—2.6 (4H, m)

Reference Example 3

Preparation of (±)-cis-7$\beta$-azido-4$\alpha$-hydroxy-1-azabicyclo[4,2,0]oct-2-en-8-on-2-carboxylic acid, t-butyl ester:

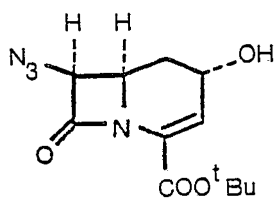

In this Example, 200 mg of (±)-cis-7-azido-1-azabicyclo[4,2,0]oct-2-en-8-on-2-carboxylic acid, t-butyl ester obtained as in Reference Example 1 or 2 is dissolved in 8.8 ml of carbon tetrachloride and 134.9 mg of N-bromosuccinimide and catalytic amount of $\alpha,\alpha'$-azobisisobutyronitrile are added. The mixture is heated under reflux for 30 minutes.

After cooling, the reaction mixture is diluted with 5 ml of chloroform and washed with 3 ml each of water and saturated sodium chloride solution. The washing is dried with anhydrous sodium sulfate and filtered.

The filtrate is concentrated to obtain an oily 4-bromo compound. The product is immediately dissolved in 10 ml of acetone and 50 mg of silver carbonate and 50 $\mu$l of water are added. The mixture is stirred at room temperature for 10 minutes. The reaction mixture is filtered and concentrated to obtain a crude product. The product is charged on a column packed with 20 g of silica gel and elution is carried out with a mixture of n-hexane and ethyl acetate (2:1).

The eluates are concentrated to obtain 86.4 mg of the desired compound as pale yellow crystals. Yield 40.7%.

Properties of the crystals are given below.

M.P.: 100.0—101.0°C

$IR\nu_{max}^{CHCl_3}$ ($cm^{-1}$): 2130, 1790, 1635, 1630

NMR ($CDCl_3$)$\delta$: 6.30 (1H, d, J=5Hz), 5.03 (1H, d, J=5.2), 4.47 (1H, m), 3.93 (1H, m), 3.20 (1H, br), 2.1—1.8 (2H, m), 1.55 (9H, s)

Reference Example 4

Preparation of (±)-cis-7β-amino-4α-hydroxy-1-azabicyclo[4,2,0]oct-2-en-8-on-2-carboxylic acid, t-butyl ester:

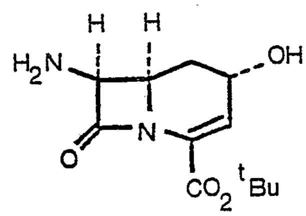

In this Example, 54.0 mg (0.19 m mole) of (±)-cis-7β-azido-4α-hydroxy-1-azabicyclo[4,2,0]oct-2-en-8-on-2-carboxylic acid, t-butyl ester prepared as in Reference Example 3 is dissolved in 20 ml of ethanol and 15 mg of 10% palladium-carbon is added. The mixture is subjected to catalytic reduction at room temperature in a stream of hydrogen for 1.5 hours and after removing the catalyst, concentrated under reduced pressure. The concentrate is subjected to silica gel chromatography (5 g of silica gel, solvent, chloroform:methanol = 4:1) to obtain 21.7 mg (44.3%) of the desired product as a yellow glass. Properties of the product are as follows.

$IR\nu_{max}^{CHCl_3}$ ($cm^{-1}$): 3250—3400, 1775, 1730, 1635

NMR ($CDCl_3$)$\delta$: 6.28 (1H, d, J=6.0Hz), 4.62 (1H, d, J=5.0Hz), 4.47 (1H, m), 4.50—3.30 (1H, m), 2.52 (3H, br), 2.50—1.50 (2H, m), 1.93 (9H, s)

Reference Example 5

Preparation of the trifluoroacetate of (±)-cis-7β-amino-4α-hydroxy-1-azabicyclo[4,2,0]oct-2-en-8-on-2-carboxylic acid:

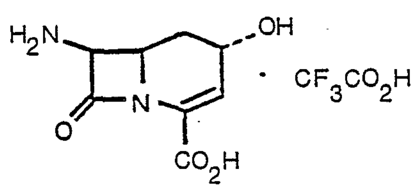

In this Example, 21.7 mg of (±)-cis-7β-amino-4α-hydroxy-1-azabicyclo[4,2,0]oct-2-en-8-on-2-carboxylic acid, t-butyl ester prepared as in Reference Example 4 is dissolved in a mixture of 1 ml of methylene chloride and 1 ml of trifluoroacetic acid and the mixture is allowed to stand at 0°C for one hour and 15 minutes. The mixture is concentrated under reduced pressure and dissolved in a mixture of 0.5 ml of methylene chloride and 1.0 ml of trifluoroacetic acid.

The solution is allowed to stand for one hour and concentrated under reduced pressure to obtain a red oily product.

The product is charged on a column packed with 5 ml of HP—20AG and elution is carried out with water to obtain ninhydrin positive fractions. The combined fractions are again charged on a column packed with 50 ml of Sephadex LH—20 and elution is carried out with water to obtain a ninhydrin positive fraction. The fraction is concentrated under reduced pressure to obtain 5.0 mg (28.6%) of the desired product.

Properties of the product are as follows.

$IR\nu_{max}^{KBr}$ ($cm^{-1}$): 3420, 2960, 1780, 1735(sh), 1620

NMR ($D_2O$ with DSS as an internal standard)$\delta$: 6.15 (1H, d, J=5.4Hz), 4.91 (1H, d, J=5.4Hz), 4.57 (1H, m), 4.08 (1H, ddd, J=12.1, 5.4, 4.0Hz), 2.22 (1H, m), 1.79 (1H, ddd — sextet apparently, J=12.1, 12.1, 4.0Hz)

Reference Example 6

Preparation of (±)-cis-7-azido-1-azabicyclo[4,2,0]oct-2-en-4,8-dion-2-carboxylic acid, t-butyl ester:

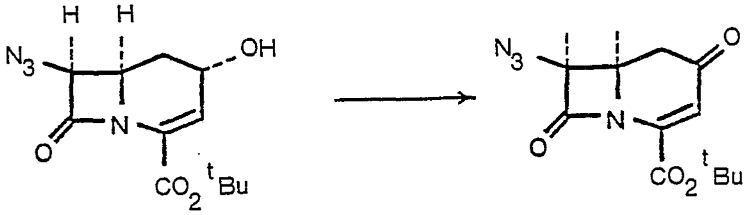

In this Example, 374 mg (2.760 m mole) of N-chlorosuccinimide is added to 7 ml of anhydrous toluene and the mixture is stirred in a stream of nitrogen at a temperature of −25°C. Then 0.35 ml (4.766 m mole) of methyl sulfide is added and the mixture is stirred for 10 minutes. After raising the temperature to −15°C, to the reaction mixture, 107 mg (0.382 m mole) of (±)-cis-7β-azido-4α-hydroxyl-1-azabicyclo[4,2,0]oct-2-en-8-on-2-carboxylic acid, t-butyl ester prepared as in Reference Example 3 in 25 ml of anhydrous toluene is added and the mixture is stirred for 2.5 hours.

A solution wherein 0.773 ml of anhydrous triethylamine is dissolved in 1.4 ml of anhydrous petroleum-ether is added to the reaction mixture in 2 minutes. The ice bath is removed and the mixture is stirred for 15 minutes. After adding 20 ml of ether, the solution is washed with a mixture of 3 ml of 5% hydrochloric acid and 20 ml of saturated saline solution and then washed with 5 ml of saturated saline solution twice. The ether layer is dried with anhydrous sodium sulfate and concentrated under reduced pressure to obtain 145.7 mg of a crude oily product of the desired compound. The product is subjected to silica gel chromatography using 10 g of Wako-gel C—200 and a solvent of n-hexane and ethyl acetate (2:1) to obtain 42.5 mg (0.153 m mole) of an oily product of the desired compound. Yield 40.1%.

Properties of the product are as follows.

$IR\nu_{max}^{CHCl_3}$ ($cm^{-1}$): 2125, 1802, 1740, 1698(sh), 1690

NMR ($CDCl_3$)$\delta$: 6.16 (1H, s), 5.22 (1H, d, J=5.5Hz), 4.44 (1H, m), 3.10—2.48 (2H, m), 1.55 (9H, s)

Reference Example 7

Preparation of (±)-cis-7β-azido-4β-hydroxy-1-azabicyclo[4,2,0]oct-2-en-8-on-2-carboxylic acid, t-butyl ester:

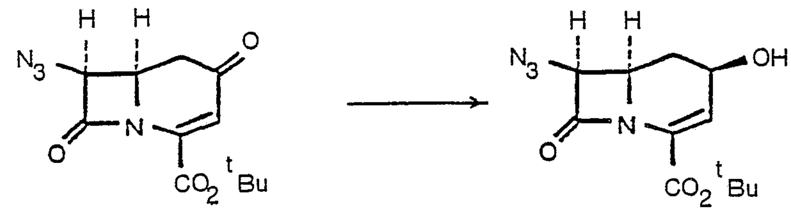

In this Example, 47 mg (0.169 m mole) of (±)-cis-7-azido-1-azabicyclo[4,2,0]oct-2-en-4,8-dion-2-carboxylic acid, t-butyl ester prepared as in Reference Example 6 is dissolved in 1.6 ml of tetrahydrofuran containing 1% of water and the solution is stirred at a temperature of −40°C. Then, 3.2 mg (0.0845 m mole) of sodium borohydride is added and the mixture is stirred for 15 minutes. After adding a mixture of 10 ml of saturated saline solution and 1 ml of 10% hydrochloric acid, the mixture is extracted with 20 ml of ether twice. The ether layer is dried with anhydrous sodium sulfate and concentrated to dryness under reduced pressure to obtain 43.9 mg (0.157 m mole) of an oily product of the desired compound. Yield 92.7%. Properties of the product are as follows.

$IR\nu_{max}^{CHCl_3}$ ($cm^{-1}$): 3450, 2118, 1789, 1784(sh), 1729(sh), 1722, 1629, 1632(sh)

NMR ($CHCl_3$)$\delta$: 6.20 (1H, d, J=1.22Hz), 4.86 (1H, d, J=5.37Hz), 4.62 (1H, m), 3.98 (1H, m), 2.45—1.65 (2H, m), 1.53 (9H, s)

Reference Example 8

Preparation of (±)-cis-7β-amino-4β-hydroxy-1-azabicyclo[4,2,0]oct-2-en-8-on-2-carboxylic acid, t-butyl ester:

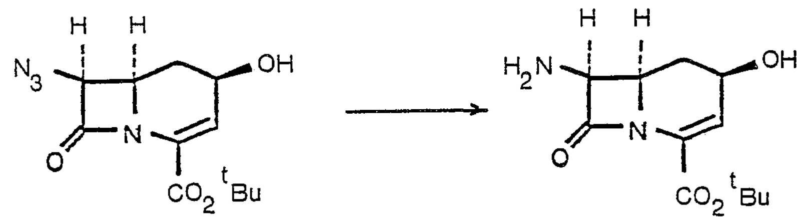

In this example, 41.9 mg (0.149 mole) of (±)-cis-7β-azido-4β-hydroxy-1-azabicyclo[4,2,0]oct-2-en-8-on-2-carboxylic acid, t-butyl ester obtained as in Reference Example 7 is dissolved in 1.5 ml of ethyl alcohol and 12 mg of 10% palladium-carbon is added. The mixture is stirred in a stream of hydrogen under ice cooling for 2 hours and 15 minutes. Then, after removing palladium-carbon by filtration, the filtrate is concentrated under reduced pressure and the concentrated residue is dissolved in 10 ml of ethyl acetate. The solution is extracted with 5 ml of 10% citric acid aqueous solution four times and the extract is washed with 5 ml of ethyl acetate. After adjusting the pH to 8 with sodium carbonate, the washing is saturated with sodium chloride and extracted with 30 ml of ethyl acetate three times. The extract is washed with 30 ml of saturated saline solution, dried with anhydrous sodium sulfate and concentrated to dryness under reduced pressure to obtain 23.4 mg (0.092 m mole) of a pale yellow

powder of the desired compound. Yield 61.6%. Properties of the compound are as follows.

$IR\nu_{max}^{CHCl_3}$ ($cm^{-1}$): 3430, 1774, 1734(sh), 1724, 1625(sh), 1618

NMR ($CDCl_3$)$\delta$: 6.18 (1H, bs), 4.53 (1H, m), 3.83 (1H, m), 2.83 (3H, bs), 2.20 (1H, m), 1.51 (9H, s)

Reference Example 9

Preparation of the trifluoroacetate of (±)-cis-7β-amino-4β-hydroxy-1-azabicyclo[4,2,0]oct-2-en-8-on-2-carboxylic acid:

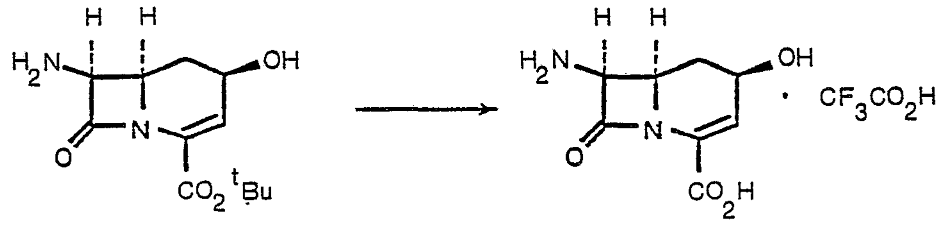

In this Example, 172.4 mg (0.678 m mole) of (±)-cis-7β-amino-4β-hydroxy-1-azabicyclo[4,2,0]oct-2-en-8-on-2-carboxylic acid, t-butyl ester obtained as in Reference Example 8 is dissolved in 8 ml of anhydrous methylene chloride and 8 ml of trifluoroacetic acid is added with stirring under ice cooling. The mixture is stirred at the same temperature for 1.5 hours. The reaction solvent is removed by distillation under reduced pressure and the resulting brown oily product is treated with ethyl ether to obtain 121.3 mg (0.389 m mole) of a yellow-brown powder. Yield 57.3%. Properties of the product are as follows.

$IR\nu_{max}^{KBr}$ ($cm^{-1}$): 3440, 1770, 1750(sh), 1700(sh), 1685

**Claims**

1. Acylated cephalosporin analogs represented by the formula I

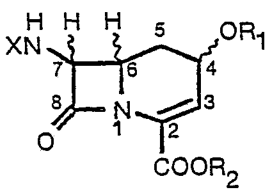

(I)

{wherein X represents an acyl group represented by the formula $X_1CO$, wherein $X_1$ represents a group selected from the following groups:

1) a group represented by the formula:

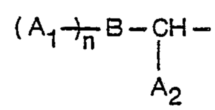

[wherein B represents a cyclohexenyl group, a cyclohexadienyl group, a phenyl group, or a five- or six-membered heterocycle selected from furyl, pyrrolyl, thiazolyl, oxazolyl, isothiazolyl, isoxazolyl, imidazolyl, pyrazolyl, triazolyl, tetrazolyl, pyridinyl, pyrimidinyl, pyrazinyl, pyridazinyl, triazinyl, 1,2,4-thiadiazolyl and 5,6-dihydro-1,4-dithiin-2-yl, $A_1$ represents substituent(s) on the ring B, which is selected from a hydrogen atom, a hydroxy group, an alkoxy group having 1 to 4 carbon, a halogen atom, a nitro group, an amino group, an aminomethyl group, a methylsulfonamido group and an acyloxy group having 1 to 4 carbon atoms, n is 0 or an integer of 1 to 5, and $A_2$ represents a hydrogen atom, an amino group, a hydroxy group, a carboxy group or a sulfo group],

2) a group represented by the formula:

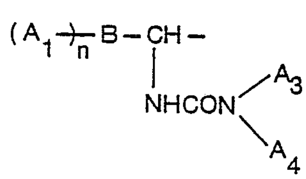

[wherein $A_1$, B and n have the same significance as defined above, $A_3$ and $A_4$ are the same or different

and represent a hydrogen atom, an alkyl group having 1 to 4 carbon atoms, a group represented by the formula

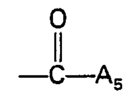

(wherein $A_5$ represents an alkyl group having 1 to 4 carbon atoms) or a group represented by the formula

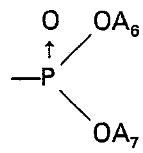

(wherein $A_6$ and $A_7$ are the same or different and represent a hydrogen atom, an alkyl group having 1 to 4 carbon atoms or an alkali metal) and

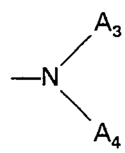

represents a group represented by the formula

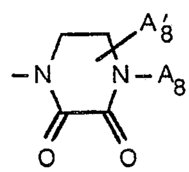

(wherein $A_8$ and $A'_8$ are the same or different and represent a hydrogen atom or an alkyl group having 1 to 4 carbon atoms) or a group represented by the formula

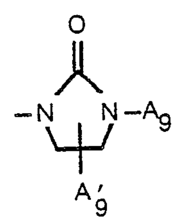

(wherein $A_9$ represents a hydrogen atom, an alkyl group having 1 to 4 carbon atoms, a methanesulfonyl group or a furfurylideneimino group, and $A'_9$ represents a hydrogen atom or an alkyl group having 1 to 4 carbon atoms)], and

3) a group represented by the formula

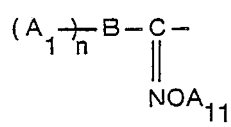

[wherein $A_1$, B and n have the same significance as defined above and $A_{11}$ represents a hydrogen atom, an alkyl group having 1 to 6 carbon atoms, an alkenyl group having 2 to 6 carbon atoms, an alkinyl gorup having 2 to 6 carbon atoms, a cycloalkyl group having 3 to 6 carbon atoms or an aryl group and those groups may be substituted by a suitable substituent selected from carboxy, cyano, halogen, carbamoyl and an alkyloxycarbonyl group having 1 to 4 carbon atoms]},

$R_1$ represents a hydrogen atom or an alkyl group having 1 to 5 carbon atoms, and

$R_2$ represents a hydrogen atom, an alkyl group having 1 to 5 carbon atoms selected from methyl, ethyl, n-propyl, isopropyl, n-butyl, isobutyl, sec-butyl and t-butyl, a halogenated alkyl group having 1 to 5 carbon atoms selected from 2,2,2-trifluoroethyl and 2,2,2-trichloroethyl, an arylmethyl group having 7 to 20 carbon atoms selected from benzyl, diphenylmethyl and triphenylmethyl, an arylmethyl group having 7 to 20 carbon atoms and having methoxy or nitro, on the phenyl ring, a substituted silyl group

selected from trimethylsilyl and triphenylsilyl or a group enzymatically or nonenzymatically readily eliminable *in vivo*, represented by the formula

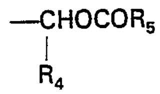

wherein $R_4$ represents a hydrogen atom or an alkyl group having 1 to 6 carbon atoms and $R_5$ represents an alkyl group having 1 to 6 carbon atoms, an alkoxy group having 1 to 6 carbon atoms or a phenyl group} and the pharmaceutically acceptable salts thereof.

2. The compounds in claim 1, wherein the hydrogens at the 6- and 7-positions have cis-configuration.

3. The compounds in claim 2, wherein $R_1$ is a hydrogen.

4. The compounds in claim 3, wherein the hydroxy group at the 4-position has cis-configuration to the hydrogens at the 6- and 7-positions.

5. The compounds in claim 3, wherein the hydroxy group at the 4-position has trans-configuration to the hydrogens at the 6- and 7-positions.

6. The compounds in claim 4, wherein X is an acyl group represented by the formula:

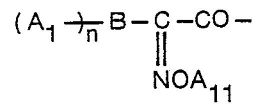

wherein $A_1$, n, B and $A_{11}$ have the same significance as defined in claim 1.

7. The compounds in claim 6, wherein the acyl group is a group represented by the formula:

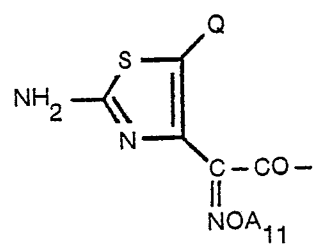

wherein $A_{11}$ has the same significance as defined in claim 1 and Q represents a hydrogen atom or a halogen atom.

8. The compounds in claim 7, wherein the —$OA_{11}$ group has syn configuration.

9. The compounds in claim 8, wherein $A_{11}$ is a methyl group.

10. The compound in claim 9, wherein $R_2$ and Q are hydrogens, that is, (±) - 7$\beta$ - [2 - (2 - amino - 4 - thiazolyl) - 2 - syn - methoxyiminoacetamido] - 4$\alpha$ - hydroxy - 1 - azabicyclo-[4,2,0]oct - 2 - en - 8 - on - 2 - carboxylic acid.

11. (4S, 6R, 7S) - 7 - [2 - (2 - amino - 4 - thiazolyl) - 2 - syn - methoxyiminoacetamido] - 4 - hydroxy - 1 - azabicyclo[4,2,0]oct - 2 - en - 8 - on - 2 - carboxylic acid.

12. The sodium salt of (4S, 6R, 7S) - 7 - [2 - (2 - amino - 4 - thiazolyl) - 2 - syn - methoxyiminoacetamido] - 4 - hydroxy - 1 - azabicyclo[4,2,0]oct - 2 - en - 8 - on - 2 - carboxylic acid.

13. The compound in claim 9, wherein $R_2$ is a hydrogen and Q is a halogen atom.

14. The compound in claim 13, wherein Q is a bromine, that is, (±) - 7$\beta$ - [2 - (2 - amino - 5 - bromo - 4 - thiazolyl) - 2 - syn - methoxyiminoacetamido] - 4$\alpha$ - hydroxy - 1 - azabicyclo-[4,2,0]oct - 2 - en - 8 - on - 2 - carboxylic acid.

15. (4S, 6R, 7S) - 7 - [2 - (2 - amino - 5 - bromo - 4 - thiazolyl) - 2 - syn - methoxyiminoacetamido] - 4 - hydroxy - 1 - azabicyclo[4,2,0]oct - 2 - en - 8 - on - 2 - carboxylic acid.

16. The compound in claim 8, wherein $A_{11}$ is a 2 - carboxy - 2 - prop - 2 - yl group, that is, (±) - 7$\beta$ - [2 - (2 - amino - 4 - thiazolyl) - 2 - syn - (2 - carboxy - 2 - prop - 2 - oxyimino)-acetamido] - 4$\alpha$ - hydroxy - 1 - azabicyclo[4,2,0]oct - 2 - en - 8 - on - 2 - carboxylic acid.

17. (4S, 6R, 7S) - 7 - [2 - (2 - amino - 4 - thiazolyl) - 2 - syn - (2 - carboxy - 2 - prop - 2 - oxyimino)acetamido] - 4 - hydroxy - 1 - azabicyclo[4,2,0]oct - 2 - en - 8 - on - 2 - carboxylic acid.

18. A process for producing cephalosporin analogs represented by the formula I according to claim 1

and the pharmaceutically acceptable salts thereof, which comprises acylating cephalosporin analogs represented by the formula V

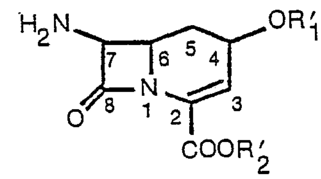

(V)

[wherein $R'_1$ is the same or different from $R_1$ and represents a hydrogen, an alkyl group having 1 to 5 carbon atoms or a protecting group of a hydroxy group and $R'_2$ is the same or different from $R_2$ and represents a hydrogen atom or a protecting group of carboxy group] or functionally equivalent compound with a carboxylic acid represented by the formula VII

$$X_2COOH \qquad (VII)$$

{wherein $X_2$ represents a group selected from the following five groups:

1') a group represented by the formula:

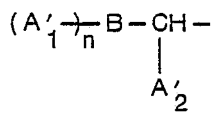

[wherein B and n have the same significance as defined above, $A'_1$ represents a substituent which is selected from a hydrogen atom, a hydroxy group, a protected hydroxy group, an alkoxy group having 1 to 4 carbon atoms, a halogen atom, a nitro group, a protected amino group, a protected aminomethyl group, a methylsulfonamido group and an acyloxy group having 1 to 4 carbon atoms, and $A'_2$ represents a hydrogen atom, a protected amino group, a hydroxy group, a protected hydroxy group, a carboxy group, a protected carboxy group, a sulfo group or a protected sulfo group],

2') a group represented by the formula:

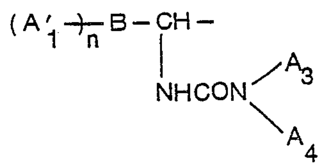

[wherein $A'_1$, $A_3$, $A_4$, B and n have the same significance as defined above], and

3') a group represented by the formula:

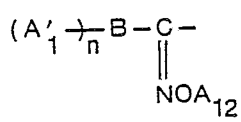

[wherein $A'_1$, B and n have the same significance as defined above and $A_{12}$ represents a hydrogen atom, an alkyl group having 1 to 6 carbon atoms, an alkenyl group having 2 to 6 carbon atoms, an alkinyl group having 2 to 6 carbon atoms, a cycloalkyl group having 3 to 6 carbon atoms or an aryl group and whose groups may be substituted with a suitable substituent which is selected from a protected carboxy group, a cyano group, a halogen atom, a carbamoyl group and a lower alkyloxycarbonyl group having 1 to 4 carbon atoms]}, or a reactive derivative thereof and, then, optionally eliminating the protecting group in the group $X_2CO$—, $OR'_1$ and/or —$COOR'_2$ and, optionally, converting to pharmaceutically acceptable salts thereof.

19. The process in claim 18, wherein the hydrogens at the 6- and 7-positions have cis-configuration.

20. The process in claim 19, wherein $R'_1$ and $R'_2$ are hydrogens.

21. The process in claim 20, wherein the hydroxy group at the 4-position has cis-configuration to the hydrogens at the 6- and 7-positions.

22. The process in claim 21, wherein the hydroxy group at the 4-position has trans-configuration to the hydrogens at the 6- and 7-positions.

23. The process for producing cephalosporin analogs represented by the formula VI (6R, 7S)

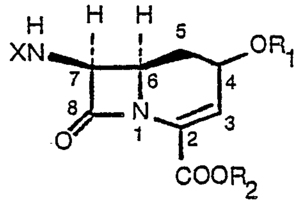

(VI)

(wherein X, $R_1$ and $R_2$ have the same significance as defined in claim 18 and pharmaceutically acceptable salts thereof, which comprises acylating cephalosporin analogs represented by the formula V-1

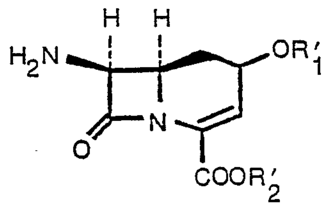

(V–1)

(wherein $R'_1$ and $R'_2$ have the same significance as defined above) or a functionally equivalent compound with a carboxylic acid represented by the formula VII

$$X_2COOH \quad (VII)$$

(wherein $X_2$ has the same significance as defined above) or a reactive derivative thereof, and then, optionally eliminating the protecting group in the group $X_2CO$—, $OR'_1$ and/or —$COOR'_2$ and optionally converting to pharmaceutically acceptable salts thereof.

24. The process in claim 23, wherein the absolute configuration at the 4-position is "S".

25. Pharmaceutical compositions with antimicrobial activity comprising a compound according to any of the claims 1 to 17.

**Patentansprüche**

1. Acylierte Cephalosporin-Analoga der Formel I

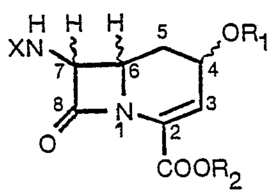

(I)

[in der X eine Acylgruppe der Formel $X_1CO$ bedeutet, worin $X_1$ eine der nachstehenden Bedeutungen hat:

1) eine Gruppe der Formel:

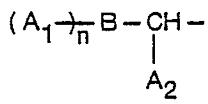

[in der B einen Cyclohexenylrest, Cyclohexadienylrest, Phenylrest oder einen 5- oder 6-gliedrigen Heterocyclus aus der Gruppe der Furyl-, Pyrrolyl-, Thiazolyl-, Oxazolyl-, Isothiazolyl-, Isoxazolyl-, Imidazolyl-, Pyrazolyl-, Triazolyl-, Tetrazolyl-, Pyridinyl-, Pyrimidinyl-, Pyrazinyl-, Pyridazinyl-, Triazinyl-, 1,2,4-Thiadiazolyl- und 5,6-Dihydro-1,4-dithiin-2-yl-Rest bedeutet, $A_1$ einen oder mehrere Substituenten am Ring B aus der nachstehenden Gruppe bedeutet:
Wasserstoffatom, Hydroxylgruppe, alkoxygruppe mit 1 bis 4 Kohlenstoffatomen, Halogenatom, Nitrogruppe, Aminogruppe, Aminomethylgruppe, Methylsulfonamidogruppe und Acyloxygruppe mit 1 bis 4 Kohlenstoffatomen; n 0 ist oder eine ganze zahl von 1 bis 5, und $A_2$ ein Wasserstoffatom, eine Aminogruppe, Hydroxylgruppe, Carboxylgruppe oder eine Sulfogruppe bedeutet]

2) eine Gruppe der Formel

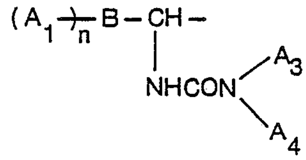

[in der $A_1$, B und n die vorstehende Bedeutung haben, $A_3$ und $A_4$ gleich oder verschieden sind und ein Wasserstoffatom, eine Alkylgruppe mit 1 bis 4 Kohlenstoffatomen, eine Gruppe der Formel

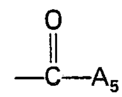

bedeutet (in der $A_5$ eine Alkylgruppe mit 1 bis 4 Kohlenstoffatomen bedeutet), oder eine Gruppe der Formel

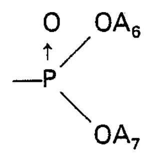

(in der $A_6$ und $A_7$ gleich oder verschieden sind und ein Wasserstoffatom, eine Alkylgruppe mit 1 bis 4 Kohlenstoffatomen oder ein Alkalimetall bedeuten), und

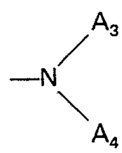

eine Gruppe der Formel

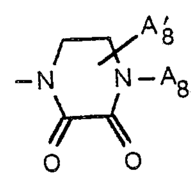

(in der $A_8$ und $A'_8$ gleich oder verschieden sind und ein Wasserstoffatom oder eine Alkylgruppe mit 1 bis 4 Kohlenstoffatomen bedeuten), oder eine Gruppe der Formel

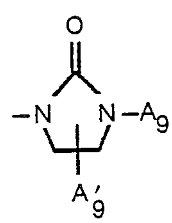

bedeutet, in der $A_9$ ein Wasserstoffatom, einen Alkylrest mit 1 bis 4 Kohlenstoffatomen, eine Methansulfonylgruppe oder eine Furfurylideniminogruppe bedeutet und $A'_9$ ein Wasserstoffatom oder einen Alkylrest mit 1 bis 4 Kohlenstoffatomen bedeutet)], und

3) eine Gruppe der Formel

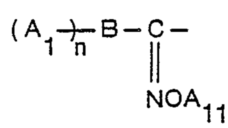

[in der $A_1$, B und n die vorstehende Bedeutung haben und $A_{11}$ ein Wasserstoffatom, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen, eine Cycloalkylgruppe mit 3 bis 6 Kohlenstoffatomen oder eine Arylgruppe bedeutet und die genannten Gruppen gegebenenfalls mit einem geeigneten Substituenten versehen

sein können, aus der nachstehenden Gruppe: Carboxy-, Cyan, Halogen-, Carbamoyl- und Alkoxycarbonylrest mit 1 bis 4 Kohlenstoffatomen]}
$R_1$ ein Wasserstoffatom oder einen Alkylrest mit 1 bis 5 Kohlenstoffatomen bedeutet und $R_2$ ein Wasserstoffatom, einen Alkylrest mit 1 bis 5 Kohlenstoffatomen, der aus der nachstehenden Gruppe gewählt ist: Methyl-, Äthyl-, n-Propyl-, Isopropyl-, n-Butyl-, Isobutyl-, sek.-Butyl und t-Butyl-Rest, einen halogenierten Alkylrest mit 1 bis 5 Kohlenstoffatomen, der aus der nachstehenden Gruppe ausgewählt ist: 2,2,2-Trifluoräthyl- und 2,2,2-Trichloräthylrest, einen Arylmethylrest mit 7 bis 20 Kohlenstoffatomen aus der Gruppe Benzyl-, Diphenylmethyl- und Triphenylmethylrest, einen Arylmethylrest mit 7 bis 20 Kohlenstoffatomen mit Methoxy- oder Nitroresten am Phenylring, einen substituierten Silylrest aus der Gruppe Trimethylsilyl- und Triphenylsilylrest oder eine enzymatisch oder nicht-enzymatisch leicht in vivo eliminierbare Gruppe der Formel

$$—\underset{\underset{R_4}{|}}{C}HOCOR_5$$

bedeutet, in der $R_4$ ein Wasserstoffatom oder einen Alkylrest mit 1 bis 6 Kohlenstoffatomen darstellt und $R_5$ einen Alkylrest mit 1 bis 6 Kohlenstoffatomen, eine Alkoxygruppe mit 1 bis 6 Kohlenstoffatomen oder einen Phenylrest bedeutet}
und ihre pharmazeutisch verträglichen Salze.

2. Verbindungen nach Anspruch 1, dadurch gekennzeichnet, daß die Wasserstoffatome in den 6- und 7-Stellungen die cis-Konfiguration aufweisen.

3. Verbindungen nach Anspruch 2, dadurch gekennzeichnet, daß $R_1$ ein Wasserstoffatom ist.

4. Verbindungen nach Anspruch 3, dadurch gekennzeichnet, daß die Hydroxylgruppe in der 4-Stellung die cis-Konfiguration bezüglich der Wasserstoffatome in den 6- und 7-Stellungen aufweist.

5. Verbindungen nach Anspruch 3, dadurch gekennzeichnet, daß die Hydroxylgruppe in der 4-Stellung die trans-Konfiguration bezüglich der Wasserstoffatome in den 6- und 7-Stellungen aufweist.

6. Verbindungen nach Anspruch 4, dadurch gekennzeichnet, daß X eine Acylgruppe der Formel:

$$(A_1 -)_n B - \underset{\underset{NOA_{11}}{\|}}{C} - CO -$$

bedeutet, in der $A_1$, n, B und $A_{11}$ die in Anspruch 1 angegebene Bedeutung haben.

7. Verbindungen nach Anspruch 6, dadurch gekennzeichnet, daß die Acylgruppe eine Gruppe der Formel:

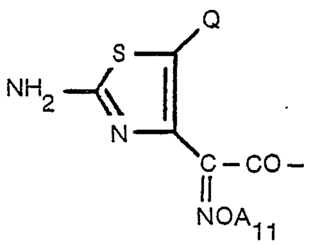

darstellt, in der $A_{11}$ die in Anspruch 1 angegebene Bedeutung hat und Q ein Wasserstoff- oder Halogenatom bedeutet.

8. Verbindungen nach Anspruch 7, dadurch gekennzeichnet, daß die Gruppe $—OA_{11}$ die syn-Konfiguration hat.

9. Verbindungen nach Anspruch 8, dadurch gekennzeichnet, daß $A_{11}$ eine Methylgruppe ist.

10. Verbindung nach Anspruch 9, in der $R_2$ und Q Wasserstoffatome bedeuten, nämlich (±) - 7β- [2-(2-Amino-4-thiazolyl)-2-syn-methoxyiminoacetamido]-4α-hydroxy-1-azabicyclo-[4,2,0]-oct-2-en-8-on-2-carbonsäure.

11. (4S, 6R, 7S)-7-[2-(2-Amino-4-thiazolyl-2-syn-methoxyiminoacetamido]-4-hydroxy-1-azabicyclo[4,2,0]-oct-2-en-8-on-2-carbonsäure.

12. Das Natriumsalz der (4S, 6R, 7S) - 7 - [2 - (2 - Amino - 4 - thiazolyl) - 2 - syn - methoxyiminoacetamido] - 4 - hydroxy - 1 azabicyclo[4,2,0] - oct - 2 - en - 8 - on - 2 - carbonsäure.

13. Verbindung nach Anspruch 9, dadurch gekennzeichnet, daß $R_2$ ein Wasserstoffatom bedeutet und Q ein Halogenatom ist.

14. Verbindung nach Anspruch 13, dadurch gekennzeichnet, daß Q ein Bromatom bedeutet, nämlich (±) - 7ß - [2 - (2 - Amino - 5 - brom - 4 - thiazolyl) - 2 - syn - methoxyiminoacetamido] - 4α - hydroxy - 1 - azabicyclo[4,2,0] - oct - 2 - en - 8 - on - 2 - carbonsäure.

15. (4S, 6R, 7S) - 7 - [2 - (2 - Amino - 5 - brom - 4 - thiazolyl) - 2 - syn - methoxyiminoacetamido] - 4 - hydroxy - 1 - azabicyclo[4,2,0] - oct - 2 - en - 8 - on - 2 - carbonsäure.

16. Verbindung nach Anspruch 8, dadurch gekennzeichnet, daß $A_{11}$ eine 2-Carboxy-2-prop-2-yl-Gruppe bedeutet, nämlich (±)-7β-[2-(2-Amino-4-thiazolyl)-2-syn-(2-carboxy-2-prop-2-oxyimino)-acetamido]-4α-hydroxy-1-azabicyclo[4,2,0]-oct-2-en-8-on-2-carbonsäure.

17. (4S, 6R, 7S)-7-[2-(2-Amino-4-thiazolyl)-2-syn-(2-carboxy-2-prop-2-oxyimino)-acetamido]-4-hydroxy-1-azabicyclo[4,2,0]-oct-2-en-8-on-2-carbonsäure.

18. Verfahren zur Herstellung von Cephalosporin-Analoga der Formel I nach Anspruch 1 sowie ihrer pharmazeutisch verträglichen Salze, dadurch gekennzeichnet, daß man Cephalosporin-Analoga der Formel V

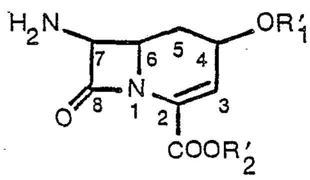

(V)

[in der $R'_1$ gleich oder verschieden von $R_1$ ist und ein Wasserstoffatom, eine Alkylgruppe mit 1 bis 5 Kohlenstoffatom oder eine Schutzgruppe der Hydroxylgruppe bedeutet und $R'_2$ gleich oder verschieden von $R_2$ ist und ein Wasserstoffatom oder eine Schutzgruppe der Carboxylgruppe darstellt] oder eine funktionelle äquivalente Verbindung mit einer Carbonsäure der Formel VII acyliert

$$X_2COOH \quad \text{(VII)}$$

{in der $X_2$ einen Rest mit einer der nachstehenden drei Bedeutungen hat:

1') eine Gruppe der Formel:

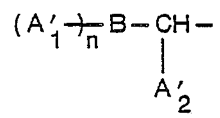

[in der B und n die vorstehende Bedeutung haben, $A'_1$ einen Rest aus der nachstehenden Gruppe bedeutet: Wasserstoffatom, Hydroxylgruppe,geschützte Hydroxylgruppe, Alkoxygruppe mit 1 bis 4 Kohlenstoffatomen, Halogenatom, Nitrogruppe, geschützte Aminogruppe, geschützte Aminomethylgruppe, Methylsulfonamidogruppe, Acyloxygruppe mit 1 bis 4 Kohlenstoffatomen, und $A'_2$ ein Wasserstoffatom, eine geschützte Aminogruppe, eine Hydroxylgruppe, eine geschützte Hydroxylgruppe, eine Carboxylgruppe, eine geschützte Carboxylgruppe, eine Sulfogruppe oder eine geschützte Sulfogruppe darstellt],

2') eine Gruppe der Formel

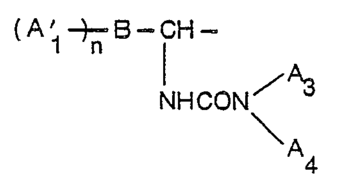

[in der $A'_1$, $A_3$, $A_4$, B und n die vorstehende Bedeutung haben], und

3') eine Gruppe der Formel

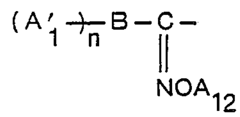

[in der $A'_1$, B und n die vorstehende Bedeutung haben und $A_{12}$ ein Wasserstoffatom, einen Alkylrest mit 1 bis 6 Kohlenstoffatomen, einen Alkenylrest mit 2 bis 6 Kohlenstoffatomen, einen Alkinylrest mit 2 bis 6 Kohlenstoffatomen, einen Cycloalkylrest mit 3 bis 6 Kohlenstoffatomen oder eine Arylgruppe bedeutet und diese Gruppen und Reste wiederum mit einem geeigneten Substituenten aus der Gruppe Carboxylrest, Cyangruppe, Halogenatom, Carbamoylgruppe, und nieder-Alkoxycarbonylrest mit 1 bis 4 Kohlenstoffatomen substituiert sein können]},

oder mit einem reaktiven Derivat der vorstehend erwähnten Carbonsäure acyliert und man anschließend gegebenenfalls aus den Resten $X_2CO$—, $OR'_1$ und/oder —$COOR'_2$ die Schutzgruppe abspal-

tet, und man gegebenenfalls das Produkt in dessen pharmazeutisch verträgliche Salze umwandelt.

19. Verfahren nach Anspruch 18, dadurch gekennzeichnet, daß die Wasserstoffatome in den 6- und 7-Stellungen die cis-Konfiguration aufweisen.

20. Verfahren nach Anspruch 19, dadurch gekennzeichnet, daß die Reste $R'_1$ und $R'_2$ Wasserstoffatome darstellen.

21. Verfahren nach Anspruch 20, dadurch gekennzeichnet, daß die Hydroxylgruppe in der 4-Stellung die cis-Konfiguration bezüglich der Wasserstoffatome in den 6- und 7-Stellungen hat.

22. Verfahren nach Anspruch 21, dadurch gekennzeichnet, daß die Hydroxylgruppe in der 4-Stellung die trans-Konfiguration bezüglich der Wasserstoffatome in den 6- und 7-Stellungen hat.

23. Verfahren zur Herstellung von Cephalosporinanaloga der Formel VI (6R, 7S)

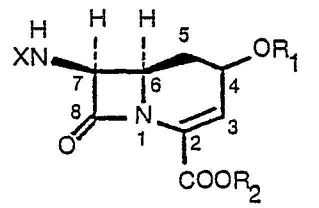

(VI)

(in der X, $R_1$ und $R_2$ die in Anspruch 18 angegebene Bedeutung haben) und ihrer pharmazeutisch verträglichen Salze, dadurch gekennzeichnet, daß man Cephalosporinanaloga der Formel V-1

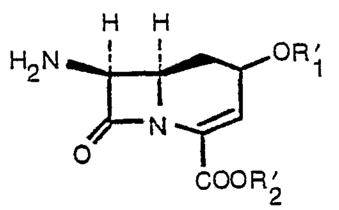

(V-1)

in der $R'_1$ und $R'_2$ die vorstehende Bedeutung haben) oder eine funktionell äquivalente Verbindung mit einer Carbonsäure der Formel VII

$$X_2COOH \quad (VII)$$

(in der $X_2$ die vorstehende Bedeutung hat) oder mit einem reaktiven Derivat dieser Verbindung acyliert, und man anschließend gegebenenfalls die Schutzgruppe an den Resten $X_2CO$—, $OR'_1$ und/oder —$COOR'_2$ abspaltet und man gegebenenfalls die Verbindung in ihre pharmazeutisch verträglichen Salze umwandelt.

24. Verfahren nach Anspruch 23, dadurch gekennzeichnet, daß die absolute Konfiguration in der 4-Stellung die S-Konfiguration ist.

25. Pharmazeutische Zubereitungen mit antimikrobieller Wirksamkeit enthaltend eine Verbindung gemäß jedem der Ansprüche 1 bis 17.

## Revendications

1. Analogues acylés de la céphalosporine, représentés par la formule I

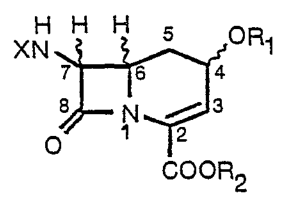

(I)

[où X représente un groupe acyle représenté par la formule $X_1CO$ où $X_1$ représente un groupe choisi parmi les groupes suivants:

1) un groupe représenté par la formule

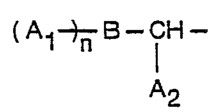

[où B représente un groupe cyclohexényle, un groupe cyclohexadiényle, un groupe phényle, ou un

hétérocycle pentagonal ou hexagonal choisi parmi les radicaux furyle, pyrrolyle, thiazolyle, oxazolyle, isothiazolyle, isoxazolyle, imidazolyle, pyrazolyle, triazolyle, tétrazolyle, pyridinyle, pyrimidinyle, pyrazinyle, pyridazinyle, triazinyle, 1,2,4-thiadiazolyle et 5,6-dihydro-1,4-dithiine-2-yle; $A_1$ représente un ou plusieurs substituants sur le noyau B, qui est choisi parmi un atome d'hydrogène, un groupe hydroxy, un groupe alcoxy à 1 à 4 atomes de carbone, un atome d'halogène, un groupe nitro, un groupe amino, un groupe aminométhyle, un groupe méthylsulfonamido et un groupe acyloxy à 1 à 4 atomes de carbone; n vaut 0 ou est un entier de 1 à 5, et $A_2$ représente un atome d'hydrogène, un groupe amino, un groupe hydroxy, un groupe carboxy ou un groupe sulfo],

2) un groupe représenté par la formule

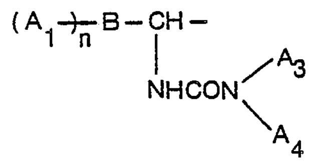

[où $A_1$, B et n ont la même signification que celle donnée ci-dessus, $A_3$ et $A_4$ sont identiques ou différents et représentent un atome d'hydrogène, un groupe alkyle à 1 à 4 atomes de carbone, un groupe représenté par la formule

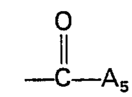

(où $A_5$ représente un groupe alkyle à 1 à 4 atomes de carbone) ou un groupe représenté par la formule

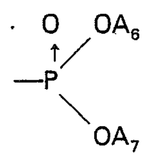

(où $A_6$ et $A_7$ sont identiques ou différents et représentent un atome d'hydrogène, un groupe alkyle à 1 à 4 atomes de carbone ou un métal alcalin) et

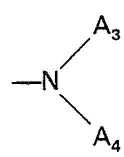

représente un groupe représenté par la formule

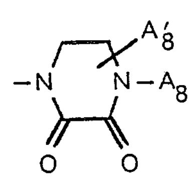

(dans laquelle $A_8$ et $A'_8$ sont identiques ou différents et représentent un atome d'hydrogène ou un groupe alkyle à 1 à 4 atomes de carbone), ou bien un groupe représenté par la formule

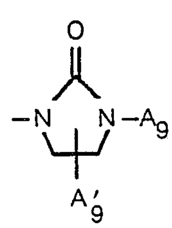

(où $A_9$ représente un atome d'hydrogène, un groupe alkyle à 1 à 4 atomes de carbone, un groupe méthanesulfonyle ou un groupe furfurylidènimino, et $A'_9$ représente un atome d'hydrogène ou un groupe alkyle à 1 à 4 atomes de carbone)], et

3) un groupe représenté par la formule

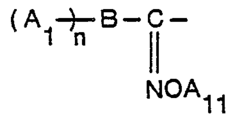

[dans laquelle $A_1$, B et n ont la même signification que celle donnée ci-dessus, et $A_{11}$ représente un atome d'hydrogène, un groupe alkyle à 1 à 6 atomes de carbone, un groupe alcényle à 2 à 6 atomes de carbone, un groupe alcinyle à 2 à 6 atomes de carbone, un groupe cycloalkyle à 3 à 6 atomes de carbone ou un groupe aryle, et ces groupes peuvent être substitués par un substituant approprié choisi parmi les radicaux carboxy, cyano, halogène, carbamoyle et alkyloxycarbonyle à 1 à 4 atomes de carbone]},
$R_1$ représente un atome d'hydrogène ou un groupe alkyle à 1 à 5 atomes de carbone, et $R_2$ représente un atome d'hydrogène, un groupe alkyle à 1 à 5 atomes de carbone choisi parmi les radicaux méthyle, éthyle, n-propyle, isopropyle, n-butyle, isobutyle, sec-butyle et tert-butyle, un radical alkyle halogéné à 1 à 5 atomes de carbone choisi parmi les radicaux 2,2,2-trifluoréthyle et 2,2,2-trichloréthyle, un groupe arylméthyle à 7 à 20 atomes de carbone choisis parmi les radicaux benzyle, diphénylméthyle et triphénylméthyle, un groupe arylméthyle à 7 à 20 atomes de carbone et possédant un radical méthoxy ou nitro sur le noyau phényle, un groupe silyle substitué choisi parmi les radicaux triméthylsilyle ou triphénylsilyle ou un groupe pouvant être facilement éliminé in vivo par voie enzymatique ou non-enzymatique, représenté par la formule

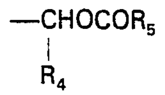

où $R_4$ représente un atome d'hydrogène ou un groupe alkyle à 1 à 6 atomes de carbone et $R_5$ représente un groupe alkyle à 1 à 6 atomes de carbone, un groupe alcoxy à 1 à 6 atomes de carbone ou un groupe phényle, et leurs sels pharmaceutiquement acceptables.

2. Composés selon la revendication 1, dans lesquels les atomes d'hydrogène sur les positions 6 et 7 ont une configuration cis.

3. Composés selon la revendication 2, dans lesquels $R_1$ est l'hydrogène.

4. Composés selon la revendication 3, dans lesquels le groupe hydroxy sur la position 4 à la configuration cis par rapport aux atomes d'hydrogène des positions 6 et 7.

5. Composés selon la revendication 3, dans lesquels le groupe hydroxy sur la position 4 est en position trans par rapport aux atomes d'hydrogène des positions 6 et 7.

6. Composés selon la revendication 4, dans lesquels X est un groupe acyle représenté par la formule

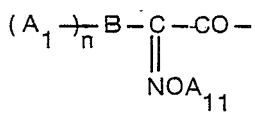

où $A_1$, n, B et $A_{11}$ ont la même signification que celle définie dans la revendication 1.

7. Composés selon la revendication 6, dans lesquels le groupe acyle est un groupe représenté par la formule

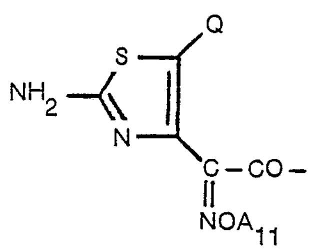

où $A_{11}$ a la même signification que celle donnée dans la revendication 1, et Q représente un atome d'hydrogène ou d'halogène.

8. Composés selon la revendication 7, dans lesquels le groupe —$OA_{11}$ a une configuration syn.

9. Composés selon la revendication 8, dans lesquels $A_{11}$ est un groupe méthyle.

10. Composé selon la revendication 9, dans lequel $R_2$ et Q sont des atomes d'hydrogène, c'est-à-dire l'acide (±) - 7ß - [2 - (2 - amino-4-thiazolyl) - 2 - syn - méthoxyiminoacétamido] - 4$\alpha$ - hydroxy - 1 - azabicyclo[4,2,0]oct - 2 - ène - 8 - one - 2 - carboxylique.

11. Acide (4S, 6R, 7S) - 7 - [2 - (2 - amino - 4 - thiazolyl) - 2 - syn - méthoxyiminoacétamido]-4 - hydroxy - 1 - azabicyclo[4,2,0]oct - 2 - ène - 8 - one - 2 - carboxylique.

12. Sel de sodium de l'acide (4S, 6R, 7S) - 7 - [2 - (2 - amino - 4 - thiazolyl) - 2 - syn - méthoxyiminoacétamido] - 4 - hydroxy - 1 - azabicyclo[4,2,0]oct - 2 - ène - 8 - one - 2 - carboxylique.

13. Composé selon la revendication 9, dans lequel $R_2$ est un atome d'hydrogène, et Q un atome d'halogène.

14. Composé selon la revendication 13, dans lequel Q est un atome de brome, c'est-à-dire l'acide (±) - 7β - [2 - (2 - amino - 5 - bromo - 4 - thiazolyl) - 2 - syn - méthoxyiminoacétamido] - 4α - hydroxy-1 - azabicyclo[4,2,0]oct - 2 - ène - 8 - one - 2 - carboxylique.

15. Acide (4S, 6R, 7S) - 7 - [2 - (2 - amino- 5 - bromo - 4 - thiazolyl) - 2 syn -méthoxyiminoacétamido] - 4 - hydroxy - 1 - azabicyclo[4,2,0]oct - 2 - ène - 8 - one - 2 - carboxylique.

16. Composé selon la revendication 8, dans lequel $A_{11}$ est un groupe 2 - carboxy - 2 - prop - 2 - yle, c'est - à - dire l'acide (±) - 7β - [2 - (2 - amino - 4 - thiazolyl) - 2 - syn - (2 - carboxy - 2 - prop - 2 - oxyimino) - acétamido] - 4α - hydroxy - 1 - azabicyclo[4,2,0]oct - 2 - ène - 8 - one - 2-carboxylique.

17. Acide (4S, 6R, 7S) - 7 - [2 - (2 - amino - 4 - thiazolyl) - 2 - syn - (2 - carboxy - 2 - prop - 2 - oxyimino)acétamido] - 4 - hydroxy - 1 - azabicyclo - [4,2,0]oct - 2 - ène - 8 - one - 2 - carboxylique.

18. Procédé pour la préparation d'analogues de la céphalosporine représentés par la formule I selon la revendication 1, et leurs sels pharmaceutiquement acceptables, qui consiste à acyler des analogues de la céphalosporine représentés par la formule V

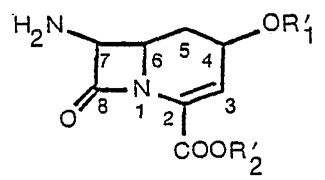

(V)

[dans laquelle $R'_1$ est identique ou différent de $R_1$ et représente un atome d'hydrogène, un groupe alkyle à 1 à 5 atomes de carbone ou un groupe protecteur d'un groupe hydroxy, et $R'_2$ est identique ou différent de $R_2$ et représente un atome d'hydrogène ou un groupe protecteur du groupe carboxy] ou un composé fonctionnellement équivalent, avec un acide carboxylique représenté par la formule VII

$$X_2COOH \qquad (VII)$$

[dans laquelle $X_2$ représente un groupe choisi parmi les cinq groupes suivants:

1') un groupe représenté par la formule:

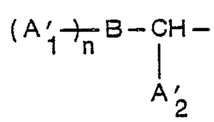

[dans laquelle B et n ont la même signification que celle donnée ci-dessus; $A'_1$ représente un substituant choisi parmi un atome d'hydrogène, un groupe hydroxy, un groupe hydroxy protégé, un groupe alcoxy à 1 à 4 atomes de carbone, un atome d'halogène, un groupe nitro, un groupe amino protégé, un groupe aminométhyle protégé, un groupe méthylsulfonamido et un groupe acyloxy à 1 à 4 atomes de carbone, et $A'_2$ représente un atome d'hydrogène, un groupe amino protégé, un groupe hydroxy, un groupe hydroxy protégé, un groupe carboxy, un groupe carboxy protégé, un groupe sulfo ou un groupe sulfo protégé],

2') un groupe représenté par la formule

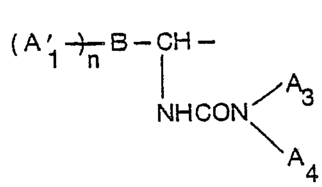

[dans laquelle $A'_1$, $A_3$, $A_4$, B et n ont la même signification que celle donnée ci-dessus], et

3') un groupe représenté par la formule

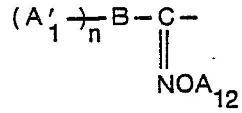

[dans laquelle $A'_1$, B et n ont la même signification que celle donnée ci-dessus, et $A_{12}$ représente un atome d'hydrogène, un groupe alkyle à 1 à 6 atomes de carbone, un groupe alcènyle à 2 à 6 atomes de carbone, un groupe alcinyle à 2 à 6 atomes de carbone, un groupe cycloalkyle à 3 à 6 atomes de carbone ou un groupe aryle et ces groupes peuvent être substitués par un substituant convenable choisi parmi un groupe carboxy protégé, un groupe cyano, un atome d'halogène, un groupe carbamoyle et un groupe alkyloxycarbonyle inférieur ayant 1 à 4 atomes de carbone]},
ou un dérivé réactif de cet acide puis, facultativement, à éliminer le groupe protecteur se trouvant dans le groupe $X_2CO$—, $OR'_1$ et/ou —$COOR'_2$ et, facultativement, à convertir en leurs sels pharmaceutiquement acceptables.

19. Procédé selon la revendication 18, dans lequel les atomes d'hydrogène sur les positions 6 et 7 ont une configuration cis.

20. Procédé selon la revendication 19, dans lequel $R'_1$ et $R'_2$ sont des atomes d'hydrogène.

21. Procédé selon la revendication 20, dans lequel le groupe hydroxy sur la position 4 a une configuration cis par rapport aux hydrogènes sur les positions 6 et 7.

22. Procédé selon la revendication 21, dans lequel le groupe hydroxy sur la position 4 possède une configuration trans par rapport aux atomes d'hydrogène des positions 6 et 7.

23. Procédé pour la production d'analogues de la céphalosporine représentés par la formule VI (6R, 7S)

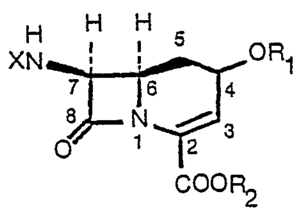

(VI)

(dans laquelle X, $R_1$ et $R_2$ ont la même signification que celle donnée dans la revendication 18) et leurs sels pharmaceutiquement acceptables, qui consiste à acyler des analogues de la céphalosporine représentés par la formule V-1

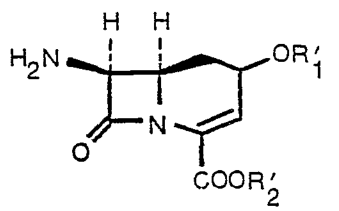

(V-1)

(dans laquelle $R'_1$ et $R'_2$ ont la même signification que celle définie ci-dessus) ou un composé fonctionnellement équivalent, avec un acide carboxylique représenté par la formule VII

$X_2COOH$ (VII)

(dans laquelle $X_2$ a la même signification que celle définie ci-dessus) ou un dérivé réactif de cet acide puis, facultativement, à éliminer le groupe protecteur dans le groupe $X_2CO$—, $OR'_1$ et/ou —$COOR'_2$ et facultativement à convertir en leurs sels pharmaceutiquement acceptables.

24. Procédé selon la revendication 23, dans lequel la configuration absolue sur la position 4 est "S".

25. Compositions pharmaceutiques à activité antimicrobienne comprenant un composé selon l'une quelconque des revendications 1 à 17.